# EUROPEAN PATENT APPLICATION

(11) **EP 2 520 566 A1**
(43) Date of publication of application: **07.11.2012**
(21) Application number: 11165173.3
(22) Date of filing: 06.05.2011
(51) Int. Cl.: C07D 207/48, C07D 401/12, A61K 31/401, A61P 25/00

(54) **New Pharmaceutical Compounds**

(71) Applicant: Orion Corporation, 02200 Espoo (FI)
(72) Inventor: The designation of the inventor has not yet been filed
(74) Representative: Sexton, Jane Helen

(57) **Abstract**

Compounds of formula I, 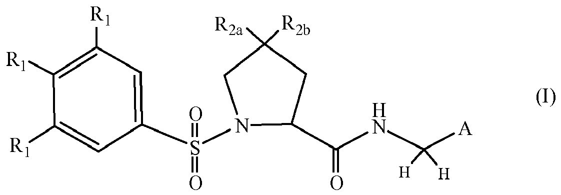 wherein A and R₁-R₇, are as defined in the claims, exhibit TRPA1 activity and are thus useful as TRPA1 modulators.

## Description

### TECHNICAL FIELD

The present invention relates to pharmacologically active proline derivatives, or pharmaceutically acceptable salts and esters thereof, as well as to pharmaceutical compositions comprising them and to their use in the treatment of diseases linked to the activation of the TRPA1 (Transient Receptor Potential subfamily A, member 1) receptors.

### BACKGROUND OF THE INVENTION

Human TRPA1 was first cloned from lung fibroblasts (Jaquemar et al., J Biol Chem 274, (1999) 7325-7333). TRPA1 was functionally characterized as a calcium permeable nonselective cation channel that is selectively localized to pain sensing peptidergic unmyelinated sensory neurons, which coexpress TRPV1, substance P and CGRP (Story et al., Cell 112 (2003) 819-829). Importantly, TRPA1 exists in both peripheral and central terminals of sensory neurons (Kosugi et al., J Neurosci 27 (2007) 4443-4451). Amino acid sequence comparison revealed that TRPA1 is a member of the transient receptor potential ion channel superfamily (Story et al., Cell 112 (2003) 819-829). A recent study finds a somewhat broader expression of TRPA1 even in myelinated fibers (Kwan et al., J Neurosci 29 (2009) 4808-4819).

Studies in healthy animals suggest that TRPA1 is not activated under physiological conditions (Wei et al., Pain 152 (2011) 582-591). Acute administration of TRPA1 agonists such as mustard oil and cinnamaldehyde to the skin causes acute pain and nocifensive behavior in healthy animals and man (Bandell et al., Neuron 41 (2004) 849-857; Namer et al., Neuroreport 16 (2005) 955-959). Several pathophysiological conditions such as acute and chronic neuropathic pain, diabetes, cancer, inflammation, asthma, arthritis, migraine, osteoarthritis, sleep deprivation, and bladder dysfunction are known to have increased production of endogenous reactive compounds such as 4-hydroxynonenal, acetaldehyde, hydrogen peroxide, prostaglandin J2, prostaglandin A2, methylglyoxal, which are known to act as TRPA1 agonists (Andersson et al., J Neurosci 28 (2008) 2485-2494; Bang et al., Eur J Neurosci 26 (2007) 2516-2523; Barnecker et al. Eur J Neurol (2011) doi:10.1111/j.1468-1331.2011.03414.x; Bessac et al., J Clin Invest 118 (2008) 1899-1910; Caceres et al., Proc Natl Acad Sci USA 106 (2009) 9099-9104; Cruz-Orengo et al., Mol Pain 4 (2008) 30; Eid et al., Mol Pain 4 (2008) 48; Fernandes et al., Arthritis Rheum 63 (2011) 819-829; Macpherson et al., J Neurosci 27 (2007) 11412-11415; Petrus et al., Mol Pain 3 (2007) 40; Premkumar et al., Soc Neurosci Abstr 40 (2010) 281.6; Taylor-Clark et al., Mol Pharmacol 73 (2008) 274-281; Thornalley, Int Rev Neurobiol 50 (2002) 37-57; Trevisani et al., Proc Natl Acad Sci USA 104 (2007) 13519-13524; Van de Bittner et al., Proc Natl Acad Sci USA 107 (2011) 21316-21321; Wei et al., Anesthesiology 111 (2009) 147-154 & Pain 152 (2011) 582-591). Interestingly, several TRPA1 agonists can be also produced through an oxidative stress-related non-enzymatic route (Hardy et al., J Lipid Res 52 (2011) 113-124; Materazzi et al. Proc Natl Acad Sci USA 105 (2008) 12045-12050). As long as endogenous TRPA1 agonist production is increased and/or TRPA1 agonist metabolism and inactivation is decreased in various disease states, TRPA1 can be expected to be activated *in vivo* (Koivisto (2011) Acta Physiologica doi: 10.1111/j.1748-1716.2011.02318.x).

TRPA1 is a nonselective cation channel with substantial calcium permeability. TRPA1 is activated through an unusual mechanism in which reactive compounds bind covalently to cysteine and lysine amino acid residues in the N-terminus of the channel protein (Hinman et al., Proc Natl Acad Sci USA 103 (2006) 19564-19568; Macpherson et al., Nature 445 (2007) 541-545). Pathophysiological sustained TRPA1 activation by reactive agonists in sensory neurons can be expected to result in axoplasmic calcium dysregulation which causes peripheral axonopathy. Axonopathy is a common diagnostic finding in chronic pain patients and patients suffering from work-related exposure to neurotoxic compounds. Axonopathy of sensory neurons is often diagnosed from diabetic patients, who suffer from chronic pain, mechanical hypersensitivity, erectile dysfunction, impaired wound healing, numbness and at later stage from leg amputations (Tesfaye in A. Veves & R.A. Malik (eds) Diabetic Neuropathy Clinical Management 2nd Edition (2007) 243-257, Humana Press, Totowa, New Jersey.).

Activation of presynaptic TRPA1 facilitates glutamate release from axon terminals of sensory neurons in the spinal cord (Kosugi et al., J Neurosci 27 (2007) 4443-4451). Enhanced glutamate release is shown to cause central pain and secondary mechanical hypersensitivity. Spontaneous pain, secondary mechanical hypersensitivity and mechanical hyperalgesia are common symptoms of neuropathic pain patients. Recently, human TRPA1 gain-of function mutation carriers were discovered and shown to have enhanced secondary hyperalgesia to peripheral TRPA1 stimulation (Kremayer et al., Neuron 66 (2010) 671-680), which confirms the role of spinal TRPA1 in processing of secondary hyperalgesia (Wei et al., Pain 152 (2011) 582-591). A recent study revealed that spinal TRPA1 plays a key role in neurogenic inflammation reflex (Wei et al., Neurosci Lett 479 (2010) 253-256), which is evoked by peripheral injury. Neurogenic inflammation is enhanced in several diseases such as fibromyalgia, migraine, complex regional pain syndromes, pain in and around the eye, and urticaria.

TRPA1 activation in the gastrointestinal tract has been shown to release serotonin from enterochromaffin cells (Nozawa et al., Proc Natl Acad Sci USA 106 (2009) 340-3413). Increased serotonin release induces hypermotility of the gut. Treatment of cancer with reactive compounds increases plasma serotonin level, which is well known to induce nausea and vomiting.

TRPA1 activation in airways has been shown to contribute to sensory neuronal hypersensitivity in several airway diseases such as chronic cough, asthma and chronic obstructive pulmonary disease (Bessac et al., J Clin Invest 118 (2008) 1899-1910; Caceres et al., Proc Natl Acad Sci USA 106 (2009) 9099-9104).

TRPA1 activation has been shown to release noradrenaline from superior cervical ganglion sympathetic neurons (Hazari et al., Environ Health Perspect Mar 4 Epub ahead of print (2011); Smith et al., Neuroreport 15 (2004) 1399-1403). Several cardiovascular disorders such as cardiac dysrhythmias and high blood pressure are well known to be caused by increased plasma noradrenaline level.

TRPA1 has been shown to play a critical role in histamine-independent itch transduction (Wilson et al., Nat Neurosci 14, (2011) 595-602).

TRPA1 activation has been shown to result in cold hypersensitivity (da Costa et al., Pain 148 (2010) 431-437; del Camino et al., J Neurosci 30 (2010) 15165-15174; Obata et al., J Clin Invest 115 (2005) 2393-2401). Cold pain is a common symptom present in several disease conditions such as dental pain, fibromyalgia, complex regional pain syndrome, cancer pain and neuropathic pain.
Selective TRPA1 modulators can be used for treatment of a large number of acute and chronic TRPA1 activation-dependent diseases and symptoms.

The International Publications WO 2009/118596, WO 2009/144548, WO 2009/147079, WO 2010/004390, WO 2010/075353, WO 2010/109287, WO 2010/109329, WO 2010/109328, WO 2010/109334, WO 2010/125469, WO 2010/132838, WO 2010/138879, WO 2010/141805 and WO 2011/043954 describe various TRPA1 modulators.

### SUMMARY OF THE INVENTION

An object of the present invention is to provide further TRPA1 modulators that can be used for the treatment of disorders, conditions or diseases mediated by TRPA1 activity. Accordingly, an object of the present invention is to provide further compounds to be used as TRPA1 modulators in the treatment of mammals. Furthermore, pharmaceutical compositions comprising the present compounds are provided.

The TRPA1 modulators of the present invention have an improved solubility and/or an improved *in vitro* metabolic stability in liver microsomes.

### DETAILED DESCRIPTION OF THE INVENTION

The present invention relates to novel TRPA1 modulators having the general formula I, 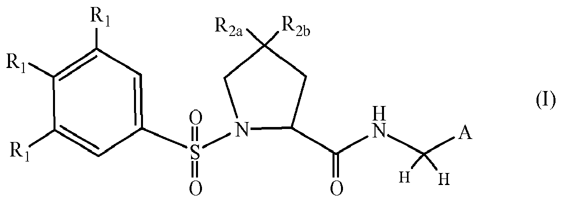 wherein
A is 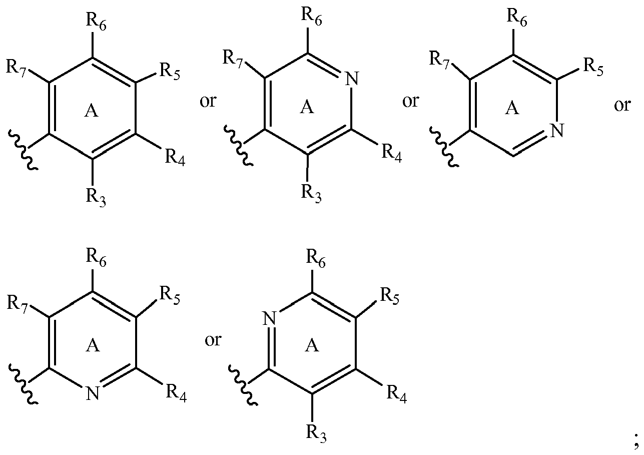 R₁ is, independently at each occurrence, H or F provided that at least one of R₁ is H;
R₂ₐ and R_{2b} are independently H or F;
R₃ is H or F;
R₄ is H, halogen, (C₁-C₆)alkyl, (C₁-C₆)alkoxy, fluoro(C₁-C₆)alkyl, fluoro(C₁-C₆)alkoxy, (C₁-C₆)alkyl-(C=O)-, (C₁-C₆)alkylamino, fluoro(C₁-C₆)alkylamino or CN;
R₅ is H, halogen, (C₁-C₄)alkyl, (C₁-C₃)alkoxy, fluoro(C₁-C₃)alkyl, fluoro(C₁-C₃)alkoxy, (C₁-C₃)alkylamino, CN or SF₅;
R₆ is H, halogen, (C₁-C₆)alkyl, cyclo(C₃-C₆)alkyl, (C₁-C₆)alkoxy,
(C₁-C₆)alkoxy(C₁-C₆)alkoxy, fluoro(C₁-C₆)alkyl, fluoro(C₁-C₆)alkoxy, (C₁-C₆)alkyl-S-, (C₁-C₆)alkyl-S-(C₁-C₆)alkoxy, (C₁-C₆)alkyl-(C=O)-, fluoro(C₁-C₆)alkyl(C=O)-, (C₁-C₆)alkylamino, fuoro(C₁-C₆)alkylamino, fluoro(C₁-C₆)alkyl-S-, CN or SF₅; and R₇ is H or F;
or a pharmaceutically acceptable salt or ester thereof;
with the provisos that the compound is not 1-((4-fluorophenyl)sulfonyl)-N-((6-methoxypyridin-3-yl)methyl)pyrrolidine-2-carboxamide, 1-((4-fluorophenyl)sulfonyl)-N-(pyridin-3-ylmethyl)pyrrolidine-2-carboxamide, 1-(phenylsulfonyl)-N-(pyridin-3-ylmethyl)pyrrolidine-2-carboxamide, N-benzyl-1-(phenylsulfonyl)pyrrolidine-2-carboxamide, N-(4-methoxybenzyl)-1-(phenylsulfonyl)pyrrolidine-2-carboxamide, N-(4-(tert-butyl)benzyl)-1-(phenylsulfonyl)pyrrolidine-2-carboxamide, N-(4-isopropylbenzyl)- I -
(phenylsulfonyl)pyrrolidine-2-carboxamide, N-(4-(difluoromethoxy)-3-methoxybenzyl)-1-(phenylsulfonyl)pyrrolidine-2-carboxamide, 1-(phenylsulfonyl)-N-(3,4,5-trimethoxybenzyl)pyrrolidine-2-carboxamide, N-benzyl-1-((4-fluorophenyl)sulfonyl)pyrrolidine-2-carboxamide, 1-((4-fluorophenyl)sulfonyl)-N-(4-methylbenzyl)pyrrolidine-2-carboxamide, N-(4-(tert-butyl)benzyl)-1-((4-fluorophenyl)sulfonyl)pyrrolidine-2-carboxamide, 1-((4-fluorophenyl)sulfonyl)-N-(4-methoxybenzyl)pyrrolidine-2-carboxamide, 1-((4-fluorophenyl)sulfonyl)-N-(3-methoxybenzyl)pyrrolidine-2-carboxamide, N-(4-fluorobenzyl)-1-((4-fluorophenyl)sulfonyl)pyrrolidine-2-carboxamide, N-(2-fluorobenzyl)-1-((4-fluorophenyl)sulfonyl)pyrrolidine-2-carboxamide, N-(3-bromobenzyl)-1-((4-fluorophenyl)sulfonyl)pyrrolidine-2-carboxamide, 1-((4-fluorophenyl)sulfonyl)-N-(4-(trifluoromethyl)benzyl)pyrrolidine-2-carboxamide, 1-((4-fluorophenyl)sulfonyl)-N-(3-(trifluoromethyl)benzyl)pyrrolidine-2-carboxamide, N-(4-(difluoromethoxy)benzyl)-1 -((4-fluorophenyl)sulfonyl)pyrrolidine-2-carboxamide or N-(4-fluoro-3-methylbenzyl)-1-((4-fluorophenyl)sulfonyl)pyrrolidine-2-carboxamide.

In a possible subgroup of the compounds of formula I, A is 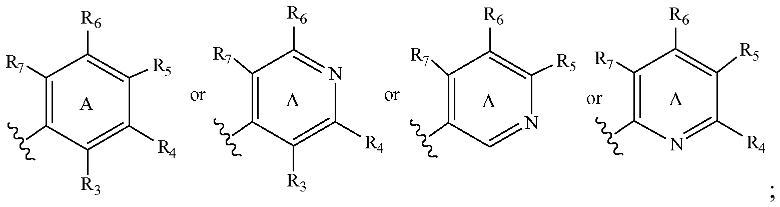 R₁ is, independently at each occurrence, H or F provided that at least one of R₁ is H;

R₂ₐ and R_{2b} are independently H or F; R₃ is H or F; R₄ is H or halogen; R₅ is H, halogen, (C₁-C₄)alkyl, fluoro(C₁-C₃)alkyl or CN; R₆ is H, halogen, (C₁-C₆)alkyl, cyclo(C₃-C₆)alkyl, (C₁-C₆)alkoxy, fluoro(C₁-C₆)alkyl, fluoro(C₁-C₆)alkoxy, (C₁-C₆)alkyl-S-, (C₁-C₆)alkyl-S-(C₁-C₆)alkoxy, (C₁-C₆)alkyl-(C=O)-, (C₁-C₆)alkylamino or SF₅; and R₇ is H; for example R₁ is, independently at each occurrence, H or F provided that at least one of R₁ is F; R₂ₐ and R_{2b} are independently H or F, for example R₂ₐ is F and R_{2b} is H; R₃ is H or F; R₄ is H or halogen; R₅ is H, halogen or fluoro(C₁-C₃)alkyl; R₆ is H, halogen, fluoro(C₁-C₆)alkyl or fluoro(C₁-C₆)alkoxy or (C₁-C₆)alkylamino; and R₇ is H.

In a further possible subgroup of the compounds of formula I, A is 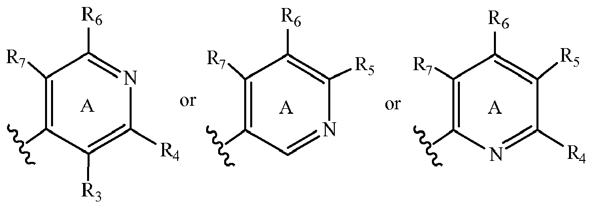 R₁ is, independently at each occurrence, H or F provided that at least one of R₁ is F, for example two of R₁ are F; R₂ₐ and R_{2b} are independently H or F; R₃ is H; R₄ is H; R₅ is H; R₆ is fluoro(C₁-C₆)alkyl or fluoro(C₁-C₆)alkoxy; and R₇ is H.

In another possible subgroup of the compounds of formula I, A is 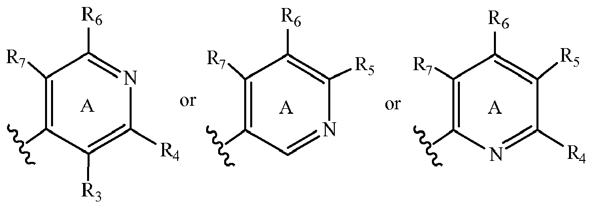 R₁ is, independently at each occurrence, H or F provided that at least one of R₁ is F, for example two of R₁ are F; R₂ₐ is F and R_{2b} is H; R₃ is H; R₄ is H; R₅ is H;
R₆ is fluoro(C₁-C₆)alkyl or fluoro(C₁-C₆)alkoxy; and R₇ is H.

In further possible subgroup of the compounds of formula I, A is 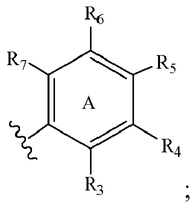 R₁ is, independently at each occurrence, H or F provided that at least one of R₁ is F, for example two of R₁ are F; R₂ₐ and R_{2b} are independently H or F; R₃ is H; R₄ is H; R₅ is fluoro(C₁-C₃)alkyl or fluoro(C₁-C₃)alkoxy; R₆ is H; and R₇ is H.

In another possible subgroup of the compounds of formula I, A is 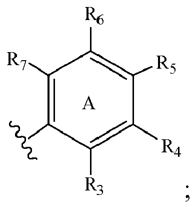 R₁ is, independently at each occurrence, H or F provided that at least one of R₁ is F, for example two of R₁ are F; R₂ₐ is F and R_{2b} is H; R₃ is H; R₄ is H; R₅ is fluoro(C₁-C₃)alkyl or fluoro(C₁-C₃)alkoxy; R₆ is H; and R₇ is H.

In further possible subgroup of the compounds of formula I, A is 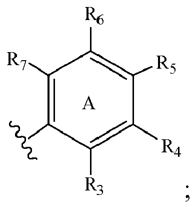 R₁ is, independently at each occurrence, H or F provided that at least one of R₁ is F, for example two of R₁ are F; R₂ₐ and R_{2b} are independently H or F; R₃ is H; R₄ is H; R₅ is H; R₆ is (C₁-C₆)alkylamino; and R₇ is H.

In another possible subgroup of the compounds of formula I, A is 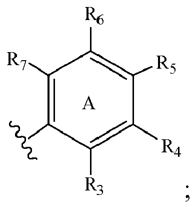 R₁ is, independently at each occurrence, H or F provided that at least one of R₁ is F, for example two of R₁ are F; R₂ₐ is F and R_{2b} is H; R₃ is H; R₄ is H; R₅ is H; R₆ is (C₁-C₆)alkylamino; and R₇ is H.

In yet another possible subgroup of the compounds of formula I, the compound is (S)-1-(4-fluorophenylsulfonyl)-N-(3-(2-(methylthio)ethoxy)benzyl) pyrrolidine-2-carboxamide, (S)-N-(3-bromobenzyl)-1-(phenylsulfonyl)pyrrolidine-2-carboxamide, (S)-N-(3-butyrylbenzyl)-1-(4-fluorophenylsulfonyl)pyrrolidine-2-carboxamide, (2S,4R)-4-fluoro-1-(4-fluorophenylsulfonyl)-N-((2-(neopentyloxy) pyridin-4-yl)methyl)pyrrolidine-2-carboxamide, (S)-N-(4-cyano-3-ethylbenzyl)-1-(4-fluorophenylsulfonyl)pyrrolidine-2-carboxamide, (S)-1-(phenylsulfonyl)-N-(3-propoxybenzyl)pyrrolidine-2-carboxamide, (S)-1-(4-fluorophenylsulfonyl)-N-((4-(2,2,2-trifluoroethoxy)pyridin-2-yl)methyl)pyrrolidine-2-carboxamide, (2S,4R)-4-fluoro-1-(4-fluorophenylsulfonyl)-N-(4-(trifluoromethyl) benzyl)pyrrolidine-2-carboxamide, (S)-N-(4-bromo-2-fluorobenzyl)-1-(4-fluorophenylsulfonyl)pyrrolidine-2-carboxamide, (S)-1-(3,5-difluorophenylsulfonyl)-N-((2-(3,3,3-trifluoropropoxy) pyridin-4-yl)methyl)pyrrolidine-2-carboxamide, (S)-N-(4-cyano-3-propylbenzyl)-1-(phenylsulfonyl)pyrrolidine-2-carboxamide, (2S,4R)-N-(4-bromo-2-fluorobenzyl)-4-fluoro-1-(4-fluorophenylsulfonyl)pyrrolidine-2-carboxamide, (S)-N-(3-(difluoromethoxy)-5-fluorobenzyl)-1-(4-fluorophenylsulfonyl) pyrrolidine-2-carboxamide, (2S,4R)-1-(3,4-difluorophenylsulfonyl)-4-fluoro-N-((2-(3,3,3-trifluoropropoxy)pyridin-4-yl)methyl)pyrrolidine-2-carboxamide, (S)-N-(3-ethoxy-5-fluorobenzyl)-1-(4-fluorophenylsulfonyl)pyrrolidine -2-carboxamide, (S)-N-(3-chloro-5-methoxybenzyl)-1-(4-fluorophenylsulfonyl) pyrrolidine-2-carboxamide, (S)-1-(phenylsulfonyl)-N-(4-(trifluoromethyl)benzyl)pyrrolidine-2-carboxamide, (2S,4R)-N-(3-(difluoromethoxy)-5-fluorobenzyl)-4-fluoro-1-(4-fluorophenylsulfonyl)pyrrolidine-2-carboxamide, (S)-1-(4-fluorophenylsulfonyl)-N-((2-(isobutylthio)pyridin-4-yl)methyl)pyrrolidine-2-carboxamide, (S)-N-(4-chlorobenzyl)-1-(4-fluorophenylsulfonyl)pyrrolidine-2-carboxamide, (2S,4R)-N-(4-chloro-3-(isopropylamino)benzyl)-4-fluoro-1-(phenylsulfonyl)pyrrolidine-2-carboxamide, (S)-N-(3-cyclopropylbenzyl)-1-(phenylsulfonyl)pyrrolidine-2-carboxamide, (S)-N-(3-cyclopropylbenzyl)-1-(4-fluorophenylsulfonyl)pyrrolidine-2-carboxamide, (S)- I - (phenylsulfonyl)-N-(3-(trifluoromethyl)benzyl)pyrrolidine-2-carboxamide, (S)-1-(phenylsulfonyl)-N-(3-pentafluorothio)benzyl)pyrrolidine-2-carboxamide, (2S,4R)-4-fluoro-1-(4-fluorophenylsulfonyl)-N-((2-(3,3,3-trifluoropropoxy)pyridin-4-yl)methyl)pyrrolidine-2-carboxamide, (S)-1-(4-fluorophenylsulfonyl)-N-((4-(trifluoromethyl)pyridin-2-yl)methyl)pyrrolidine-2-carboxamide, (S)-1-(4-fluorophenylsulfonyl)-N-((5-(isopentyloxy)pyridin-3-yl)methyl) pyrrolidine-2-carboxamide, (2S,4R)-4-fluoro-1-(4-fluorophenylsulfonyl)-N-((5-(isopentyloxy) pyridine-3-yl)methyl)pyrrolidine-2-carboxamide, (S)-1-(4-fluorophenylsulfonyl)-N-((4-isobutoxypyridin-2-yl)methyl) pyrrolidine-2-carboxamide, (S)-1-(4-fluorophenylsulfonyl)-N-((4-(isobutylthio)pyridin-2-yl)methyl) pyrrolidine-2-carboxamide, (S)-1-(3,4-difluorophenylsulfonyl)-N-((4-(2,2,2-trifluoroethoxy) pyridin-2-yl)methyl)pyrrolidine-2-carboxamide, (S)-1-(3,5-difluorophenylsulfonyl)-N-((4-(2,2,2-trifluoroethoxy) pyridine-2-yl)methyl)pyrrolidine-2-carboxamide, (2S,4R)-N-(4-cyano-3-propylbenzyl)-4-fluoro-1-(phenylsulfonyl) pyrrolidine-2-carboxamide, (S)-N-(4-cyano-3-isopropylbenzyl)-1-(phenylsulfonyl)pyrrolidine-2-carboxamide, (2S,4R)-4-fluoro-1-(4-fluorophenylsulfonyl)-N-((2-(isobutylthio) pyridin-4-yl)methyl)pyrrolidine-2-carboxamide, (S)-N-(4-bromobenzyl)-1-(4-fluorophenylsulfonyl)pyrrolidine-2-carboxamide, (S)-N-(4-ethylbenzyl)-1-(4-fluorophenylsulfonyl)pyrrolidine-2-carboxamide or (S)-1-(4-fluorophenylsulfonyl)-N-(3-(propylamino)benzyl)pyrrolidine-2-carboxamide.

The terms employed herein have the meanings indicated below. The term "at least one" employed in the meanings below refers to one or several, such as one. For example, the term "at least one fluorine" refers to one or several fluorines, for example three, two or one fluorines, such as three fluorines.

The term "halo" or "halogen", as employed herein as such or as part of another group, refers to fluorine, chlorine, bromine or iodine.

The term "(C₁-C₃)alkyl", as employed herein as such or as part of another group, refers to a saturated hydrocarbon group having straight or branched moiety and containing 1, 2, or 3 carbon atom(s). Representative examples of (C₁-C₃)alkyl include, but are not limited to, methyl, ethyl, *n*-propyl and *iso*-propyl,

The term "(C₁-C₄)alkyl", as employed herein as such or as part of another group, refers to a saturated hydrocarbon group having straight or branched moiety and containing 1, 2, 3, or 4 carbon atom(s). Representative examples of (C₁-C₄)alkyl include, but are not limited to, methyl, ethyl, *n*-propyl, *iso*-propyl, *n*-butyl, *iso*-butyl, *sec*-butyl and *tert*-butyl.

The term "(C₁-C₆)alkyl", as employed herein as such or as part of another group, refers to a saturated hydrocarbon group having straight or branched moiety and containing 1, 2, 3, 4, 5 or 6 carbon atom(s). Representative examples of (C₁-C₆)alkyl include, but are not limited to, methyl, ethyl, *n*-propyl, *iso*-propyl, *n*-butyl, *iso*-butyl, *sec*-butyl, *tert*-butyl, *n*-pentyl, *iso*-pentyl, and *n*-hexyl.

The term "cyclo(C₃-C₆)alkyl" as employed herein as such or as part of another group, refers to a saturated hydrocarbon group having cyclic moiety and containing 3, 4, 5 or 6 carbon atom(s). Representative examples of cyclo(C₃-C₆)alkyl include, but are not limited to cyclopropyl, cyclobutyl, cyclopentyl and cyclohexyl.

The term "(C₁-C₃)alkoxy", as employed herein as such or as part of another group, refers to an (C₁-C₃)alkyl group, as defined herein, appended to the parent molecular moiety through an oxygen atom. Representative examples of (C₁-C₃)alkoxy include, but are not limited to, methoxy, ethoxy and *n*-propoxy.

The term "(C₁-C₆)alkoxy", as employed herein as such or as part of another group, refers to an (C₁-C₆)alkyl group, as defined herein, appended to the parent molecular moiety through an oxygen atom. Representative examples of (C₁-C₆)alkoxy include, but are not limited to, methoxy, ethoxy, *n*-propoxy, *n*-butoxy, *iso*-butoxy, *sec*-butoxy, *tert*-butoxy, 2,2-dimethylpropoxy, 3-methylbutoxy, and n-hexoxy.

The term "fluoro(C₁-C₃)alkyl" or "fluoro(C₁-C₆)alkyl", as employed herein as such or as part of another group, refers to at least one fluorine appended to the parent molecular moiety through an (C₁-C₃)alkyl or (C₁-C₆)alkyl group, as defined herein. When there are several fluorines, the fluorines can be attached to the same or different carbon atom. Representative examples of fluoro(C₁-C₃)alkyl or fluoro(C₁-C₆)alkyl include, but are not limited to, fluoromethyl, difluoromethyl, trifluoromethyl, 2-fluoroethyl, 2,2,2-trifluoroethyl, 1,2,2-trifluoroethyl, 3-fluoropropyl, 1,3-difluoropropyl and 3,3,3-trifluoropropyl.

The term "fluoro(C₁-C₃)alkoxy" or "fluoro(C₁-C₆)alkoxy", as employed herein as such or as part of another group, refers to at least one fluorine appended to the parent molecular moiety through an (C₁-C₃)alkoxy or (C₁-C₆)alkoxy group, as defined herein. When there are several fluorines, the fluorines can be attached to the same or different carbon atom. Representative examples of fluoro(C₁-C₃)alkoxy or fluoro(C₁-C₆)alkoxy include, but are not limited to, fluoromethoxy, difluoromethoxy, trifluoromethoxy, 2-fluoroethoxy, 2,2,2-trifluoroethoxy, 3-fluoropropoxy, 3,3,3-trifluoropropoxy and 4-fluorobutoxy.

The term "amino", as employed herein as part of another group, refers to a -NH₂ group.

The term "(C₁-C₃)alkylamino", as employed herein, refers to one or two (C₁-C₃)alkyl group(s), as defined herein, appended to the parent molecular moiety through an amino group, as defined herein. When there are two (C₁-C₃)alkyl groups, the (C₁-C₃)alkyl groups can be identical or different. Representative examples of (C₁-C₃)alkylamino include, but are not limited to, *N*-methylamino, *N*-ethylamino, *N*,*N*-dimethylamino, *N,N*-diethylamino and *N-*methyl-*N*-ethylamino.

The term "(C₁-C₆)alkylamino", as employed herein, refers to one or two (C₁-C₆)alkyl group(s), as defined herein, appended to the parent molecular moiety through an amino group, as defined herein. When there are two (C₁-C₆)alkyl groups, the (C₁-C₆)alkyl groups can be identical or different. Representative examples of (C₁-C₆)alkylamino include, but are not limited to, *N*-methylamino, *N*-ethylamino, *N*-butylamino, *N*,*N*-dimethylamino and *N*,*N*-diethylamino

The term "(C₁-C₆)alkoxy(C₁-C₆)alkoxy", as employed herein as such or as part of another group, refers to at least one (C₁-C₆)alkoxy group, as defined herein, appended to the parent molecular moiety through an (C₁-C₆)alkoxy group, as defined herein. The (C₁-C₆)alkoxy groups can be identical or different. Representative examples of (C₁-C₆)alkoxy(C₁-C₆)alkoxy include, but are not limited to, methoxymethoxy, propoxymethoxy, 2-methoxyethoxy, 2-ethoxyethoxy, 2-butoxyethoxy, 2,2-dimethoxyethoxy, 1-methyl-2-propoxyethoxy, 2-methoxypropoxy and 4-methoxybutoxy.

The expression "compounds of the invention" as employed herein refers to the compounds of formula I.

The "pharmaceutically acceptable salts" according to the invention include therapeutically active, non-toxic base and and acid salt forms, which the compounds of formula I are able to form with both organic and inorganic bases and acids. Representative examples of pharmaceutically acceptable base addition salt forms, e.g. metal or amine salts, include, but are not limited to, ammonium salts, lithium, sodium, potassium, calcium, magnesium, aluminum and zinc salts, salts with organic bases, e.g. N-methyl-D-glucamine, hydrabamine salts and salts with amino acids, e.g. arginine, lysine and the like. Representative examples of pharmaceutically acceptable acid addition salts include, but are not limited to, chlorides, bromides, sulfates, nitrates, phosphates, sulfonates, methane sulfonates, formates, tartrates, maleates, citrates, benzoates, salicylates, ascorbates, acetates and oxalates, fumarates and succinates.

Pharmaceutically acceptable esters, when applicable, may be prepared by known methods using pharmaceutically acceptable acids that are conventional in the field of pharmaceuticals and that retain the pharmacological properties of the free form. Non-limiting examples of these esters include esters of aliphatic or aromatic alcohols. Representative examples of pharmaceutically acceptable esters include, but are not limited to, methyl, ethyl, *n*-propyl, *iso*-propyl, *n*-butyl, *iso*-butyl, *sec*-butyl, *tert*-butyl, and benzyl esters.

The invention includes within its scope all the possible geometric isomers, e.g. Z and E isomers (*cis* and *trans* isomers), of the compounds as well as all the possible optical isomers, e.g. diastereomers and enantiomers, of the compounds. Furthermore, the invention includes in its scope both the individual isomers and any mixtures thereof, e.g. racemic mixtures. The individual isomers may be obtained using the corresponding isomeric forms of the starting material or they may be separated after the preparation of the end compound according to conventional separation methods. For the separation of optical isomers, e.g. enantiomers, from the mixture thereof, conventional resolution methods, e.g. fractional crystallization or preparative chiral chromatography, may be used.

The compounds of formula I can be prepared by a variety of synthetic routes analogously to or according to methods known in the literature using suitable starting materials. Referring to Scheme 1, the compounds of formula I can be prepared via Intermediate 1, which is prepared from L-proline or a substituted L-proline analog of formula A and a suitable aryl-sulfonylchloride of formula B. Alternatively, an ester of L-profine or an L-proline analog (formula D) is sulfonylated with a suitable aryl-sulfonylchloride of formula B and subsequently hydrolysed to give Intermediate 1. Intermediate 1 can be reacted with a suitable amine of formula C, using some of the known amide coupling methods or by converting the carboxylic acid to the corresponding acylchloride, to form the amides of formula 1. 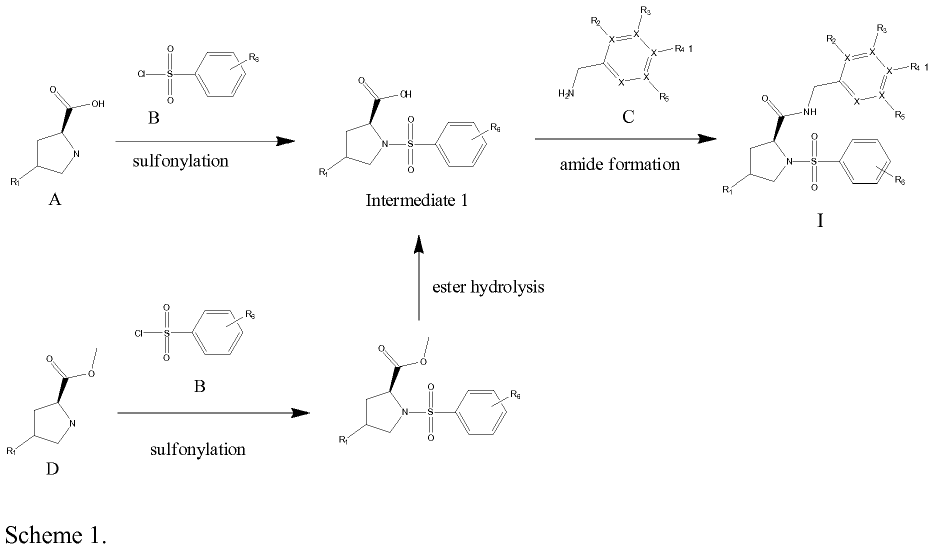 Following an alternative route 2, as described in Scheme 2, compounds of formula I can be prepared via intermediate 2 starting from an N-protected L-proline analog of formula E. A suitable protecting group is, for example, the BOC-group. The compound of formula E and a suitable amine C can be coupled to the corresponding amides using some of the known methods. Subsequent deprotection gives amine compounds like Intermediate 2. Intermediate 2 can be sulfonylated using a suitable aryl-sulfonylchloride of formula B.

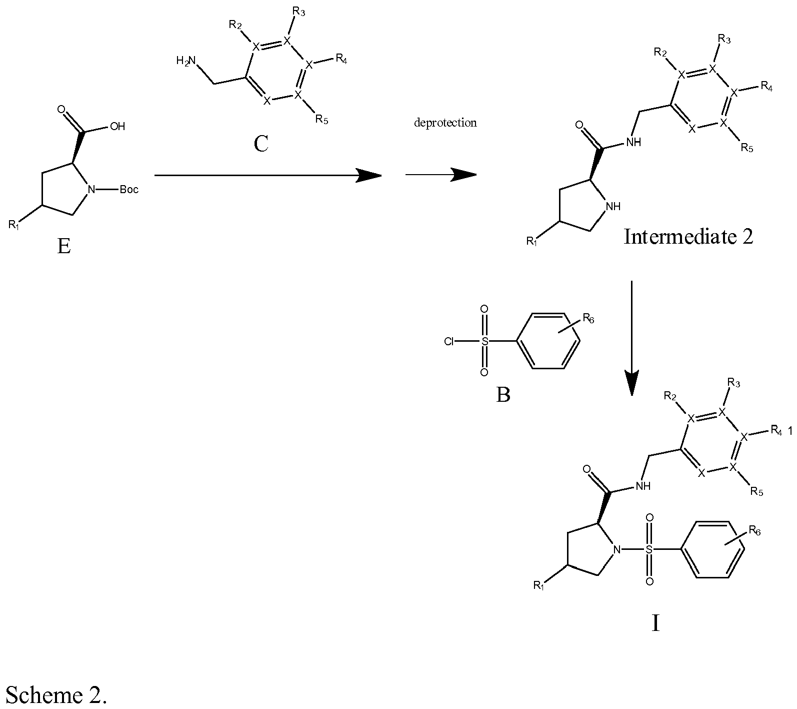

The starting materials depicted above (schemes 1 and 2) of formulae A, B, D and E are commercially available or can be prepared *via* synthetic routes known in the literature.

Suitable amines of formula C are commercially available or can be prepared by a variety of synthetic routes known in the literature, as illustrated in Scheme 3. For example, the methods include those listed below:
I. Reduction of a suitable azide, using for example hydrogenation, Staudinger reaction conditions or a hydride reagent as LiAlH₄ or NaBH₄.
II. A suitable aldehyde can be converted to the corresponding hydroxylamine and subsequently reduced using, for example, a hydride reagent as LiAlH₄.
III. Reductive amination conditions using a suitable aldehyde and ammonia.
IV. Conversion of a suitable halide or sulfonate by direct SN₂-replacement with ammonia, or using the Gabriel amine synthesis route or any similar methodology.
V. Reduction of a suitable primary amide, using a hydride reagent.
VI. Reduction of a suitable cyano-group containing compound using a hydride reagent as LiAlH₄ or NaBH₄.

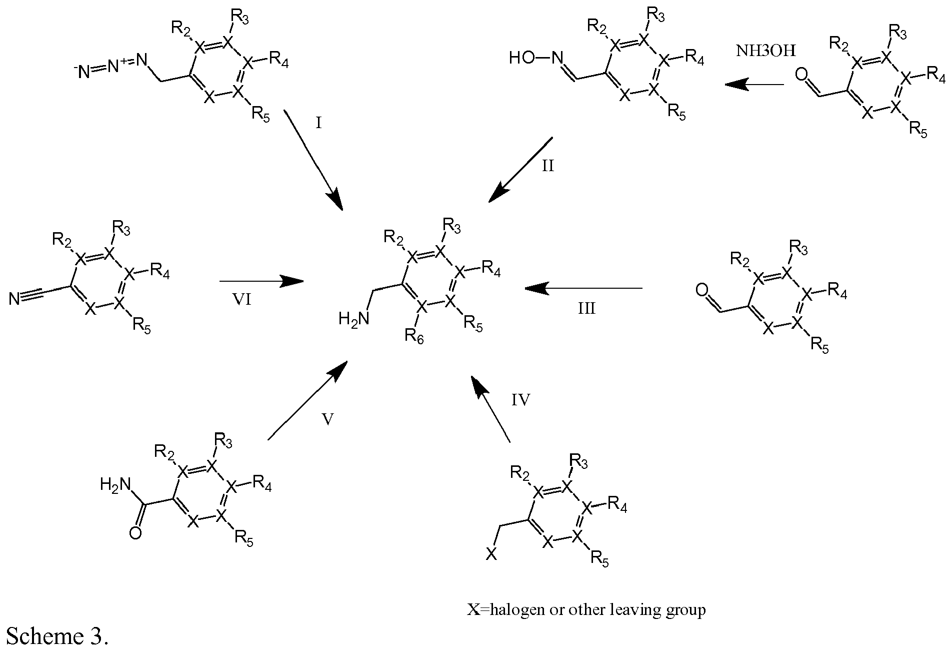

The starting materials for the reactions in Scheme 3 can be prepared using methods described in the literature.

A person skilled in the art realizes that any starting material or intermediate in the reactions described above can be protected, if necessary, in a manner known in the art. Any protected functionality can subsequently be deprotected in a manner known in the art.

The synthetic routes described above are meant to illustrate the preparation of the compounds of formula I and the preparation is by no means limited thereto, i.e. there are also other possible synthetic methods which are within the general knowledge of a person skilled in the art.

The compounds of formula I may be converted, if desired, into their pharmaceutically acceptable salt or ester form using methods known in the art.

The present invention will be explained in more detail by the following examples. The examples are meant for illustrating purposes only and do not limit the scope of the invention defined in the claims.

Column chromatography was performed on Silica gel 60 obtained from Merck, or using CombiFlash instruments together with disposable Redisep columns (Teledyne ISCO). Preparative HPLC purifications were performed with a Waters preparative HPLC/MS autopurification system equipped with a XBridge Prep C18 (5µm, 30 x 150 mm) column. Typically, a gradient of water/acetonitrile with 0.1 % formic acid was used as eluent. Microwave heating was performed using microwave reactors from Biotage. The structures of the products were confirmed by ¹H NMR. The spectra were measured with a Bruker Avance 400 instrument. LC-MS analyses were performed using a Waters Acquity UPLC/MS with an SQD or TQ detector, a Waters 2690 Micromass ZQ4000 or an Agilent 1100 Series LC/MS instrument. Continous flow hydrogenation reactions were performed using H-Cube hydrogenation reactors (ThalesNano).

### Preparation of intermediates

### Intermediate 1: (S)-1-(Phenylsulfonyl)pyrrolidine-2-carboxylic acid

(S)-Pyrrolidine-2-carboxylic acid (10.0 g, 87 mmol) was dissolved in water (200 ml). Sodium carbonate (18.4 g, 174 mmol) was added. Benzenesulfonyl chloride (11.1 ml, 87 mmol) was added dropwise. The mixture was stirred overnight and acidified with 1 M HCl and extracted three times with EtOAc. The organic layers were combined, washed with water and brine, dried over Na₂SO₄ and evaporated to dryness. This gave 22.1 g of the title compound as a white solid.
¹H NMR (400 MHz, DMSO-*d*₆) δ ppm 1.50 - 1.62 (m, 1 H), 1.73 - 1.95 (m, 3 H), 3.11 - 3.21 (m, 1 H), 3.24 - 3.48 (m, 1 H), 4.08 - 4.16 (m, 1 H), 7.58 - 7.66 (m, 2 H), 7.67 - 7.74 (m, 1 H), 7.79 - 7.89 (m, 2 H), 12.49 - 13.02 (bs, 1 H).

### Intermediate 2: (S)-1-(4-Fluorophenylsulfonyl)pyrrolidine-2-carboxylic acid

was prepared using a procedure similar to the procedure described for Intermediate 1, starting from (S)-pyrrolidine-2-carboxylic acid (10.0 g, 87 mmol,) and 4-fluorobenzenesulfonyl chloride (16.9 g, 87 mmol). 19.2 g of the title compound was obtained.
¹H NMR (400 MHz, DMSO-*d₆*) δ ppm 1.53 - 1.71 (m, 1 H), 1.73 - 2.06 (m, 3 H), 3.12-3.25 (m, 1 H), 3.30 - 3.43 (m, 1 H), 4.14 (dd, 1 H), 7.41 - 7.51 (m, 2 H), 7.87 - 7.97 (m, 2 H), 12.65 (bs, 1 H).

### Intermediate 3: (2S,4R)-4-Fluoro-1-(phenylsulfonyl)pyrrolidine-2-carboxylic acid

### Step 1: (2S,4R)-Methyl 4-fluoro-1-(phenylsulfonyl)pyrrolidine-2-carboxylate

(2S,4R)-Methyl 4-fluoropyrrolidine-2-carboxylate (3.0 g, 20.4 mmol) was dissolved in pyridine (30 ml) and the mixture was cooled on an ice-bath. Benzenesulfonyl chloride (2.60 ml, 20.4 mmol) was added slowly and the mixture was stirred for 2 h at r.t. The pyridine was removed by evaporation. 1 M HCl (50 ml) was added and the mixture was extracted three times with EtOAc, the organic layers were pooled, dried (Na₂SO₄), washed with brine and evaporated to dryness. 4.64 g of the title compound was obtained.
¹H NMR (400 MHz, CDCl₃) δ ppm 2.07 - 2.28 (m, 1 H), 2.42 - 2.59 (m, 1 H), 3.60 - 3.85 (m, 5 H), 4.33 (dd, 1 H), 5.02 - 5.24 (m, 1 H), 7.49 - 7.58 (m, 2 H), 7.58 - 7.66 (m, 1 H), 7.82 - 7.92 (m, 2 H).

### Step 2: (2S,4R)-4-Fluoro-1-(phenylsulfonyl)pyrrolidine-2-carboxylic acid

(2S,4R)-Methyl 4-fluoro-1-(phenylsulfonyl)pyrrolidine-2-carboxylate (4.64 g, 16.15 mmol) was dissolved in methanol (80 ml) and water (20 ml). Potassium hydroxide (2.72 g, 48.4 mmol) was added. The mixture was stirred until complete conversion. The mixture was acidified with 2 M HCl. The methanol was removed by evaporation. The remaining aqueous phase was extracted twice with EtOAc. The organic layers were pooled, dried (Na₂SO₄) and evaporated to dryness to give 4.61 g of the title compound.
¹H NMR (400 MHz, DMSO-*d*₆) δ ppm 2.00-2.21 (m, 1 H), 2.37-2.48 (m, 1 H), 3.51 - 3.67 (m, 2 H), 4.09 (dd, 1 H), 5.07 - 5.30 (m, 1 H), 7.56 - 7.65 (m, 2 H), 7.66 - 7.74 (m, 1 H), 7.78 - 7.88 (m, 2 H), 12.62 (bs, 1 H).

### Intermediate 4: (2S,4R)-4-Fluoro-1-(4-fluorophenylsulfonyl)pyrrolidine-2-carboxylic acid

### Step 1: (2S,4R)-Methyl 4-fluoro-1-(4-fluorophenylsulfonyl)pyrrolidine-2-carboxylate.

was prepared using a procedure similar to the procedure described for Intermediate 3, step 1, starting from (2S,4R)-methyl 4-fluoropyrrolidine-2-carboxylate hydrochloride (2.1 g, 11.4 mmol) and 4-fluorobenzenesulfonyl chloride (2.2 g, 11.4 mmol). The reaction gave 3.3 g of the title compound.
¹H NMR (400 MHz, CDCl₃) δ ppm 2.09 - 2.28 (m, 1 H), 2.47 - 2.61 (m, 1 H), 3.60 - 3.85 (m, 5 H), 4.34 (dd, 1 H), 5.06 - 5.25 (m, 1 H), 7.17 - 7.25 (m, 2 H), 7.87 - 7.95 (m, 2 H).

### Step 2: (2S,4R)-4-Fluoro-1-(4-fluorophenylsulfonyl)pyrrolidine-2-carboxylic acid

was prepared using a procedure similar to the procedure described for Intermediate 3, step 2, starting from (2S,4R)-methyl 4-fluoro-1-(4-fluorophenylsulfonyl)pyrrolidine-2-carboxylate (8.5 g, 27.8 mmol) and potassium hydroxide (3.12 g, 55.7 mmol). The reaction gave 6.13 g of the title compound.
¹H NMR (400 MHz, DMSO-*d*₆) δ ppm 2.00-2.21 (m, 1 H), 2.31-2.69 (m, 1 H), 3.48-3.73 (m, 2 H), 4.11 (dd, 1 H), 5.10-5.30 (m, 1 H), 7.40-7.50 (m, 2 H), 7.85-7.97 (m, 2 H), 12.96 (bs, 1 H).

### Intermediate 5: (2S,4R)-1-(3,4-difluorophenylsulfonyl)-4-fluoropyrrolidine-2-carboxylic acid

### Step 1: (2S,4R)-methyl 1-(3,4-difluorophenylsulfonyl)-4-fluoropyrrolidine-2-carboxylate

was prepared using a procedure similar to the procedure described for Intermediate 3, step 1, starting from (2S,4R)-methyl 4-fluoropyrrolidine-2-carboxylate hydrochloride (4.20 mmol, 1.028 g) and 3,4-difluorobenzene-1-sulfonyl chloride (4.62 mmol, 0.982 g). The product was purified by flash chromatography. 1.31 g of the title compound was obtained.

¹H NMR (400 MHz, CDCl₃) δ ppm 2.08 - 2.27 (m, 1 H) 2.50 - 2.63 (m, 1 H) 3.58 - 3.74 (m, 1 H)3.74-3.85 (m, 4 H) 4.33 - 4.39 (m, 1 H) 5.07 - 5.25 (m, 1 H) 7.29 - 7.37 (m, 1 H) 7.64 - 7.70 (m, 1 H) 7.71 - 7.77 (m, 1 H).

### Step 2: (2S,4R)-1-(3,4-Difluorophenylsulfonyl)-4-fluoropyrrolidine-2-carboxylic acid

(2S,4R)-Methyl 1-(3,4-difluorophenylsulfonyl)-4-fluoropyrrolidine-2-carboxylate (4.06 mmol, 1.314 g), tetrahydrofuran (15 ml) and water (4 ml) were stirred on an ice-bath. Lithium hydroxide (8.13 mmol, 0.195 g) was added slowly and the mixture was stirred for 2 hours. The solvent was evaporated and the residue was diluted with water and washed with dichloromethane. The aqueous phase was acidified with 5 M HCl and extracted three times with dichloromethane. The combined organic phases were evaporated to dryness. 1.22 g of the title compound was obtained.
¹H NMR (400 MHz, CDCl₃) δ ppm 2.17-2.37 (m, 1 H) 2.56-2.71 (m, 1 H) 3.60-3.87 (m, 2 H) 4.37 (dd, 1 H) 5.08-5.27 (m, 1 H) 7.30-7.39 (m, 1 H) 7.65-7.70 (m, 1 H) 7.71-7.77 (m, 1 H) 9.41 (bs, 1 H).

### Intermediate 6: (3-(2-(Methylthio)ethoxy)phenyl)methanamine

### Step 1: 3-(2-(Methylthio)ethoxy)benzonitrile

3-Cyanophenol (2.52 mmol, 0.3 g), 2-chloroethyl methyl sulfide (3.78 mmol, 0.418 g) and sodium hydroxide pellets (3.78 mmol, 0.151 g) in ethanol (4 ml) were heated at 130 °C for 30 minutes using a microwave reactor. The solvent was evaporated, the residue dissolved in ethyl acetate and washed with water and 1M NaOH. The organic layer was dried (Na₂SO₄), filtered and evaporated to dryness. The product was purified by flash chromatography. 0.487 g of the title compound was obtained.
¹H NMR (400 MHz, CDCl₃) δ ppm 2.22 (s, 3 H), 2.90 (t, 2 H), 4.17 (t, 2 H), 7.10 - 7.18 (m, 2 H), 7.22 - 7.29 (m, 1 H), 7.33 - 7.43 (m, 1 H).

### Step 2: (3-(2-(Methylthio)ethoxy)phenyl)methanamine

Lithium aluminum hydride (2.68 mmol, 0.102 g) in dry tetrahydrofuran (4 ml) was cooled on an ice bath under a nitrogen atmosphere. 3-(2-(Methylthio)ethoxy)benzonitrile (1.785 mmol, 0.345 g) in dry tetrahydrofuran (3 ml) was added dropwise. The ice bath was removed and the mixture was stirred at room temperature for 3 hours. The reaction mixture was again cooled on an ice bath. 1M NaOH was added. The mixture was stirred for an hour. The precipitate was filtered off. The filtrate was concentrated and re-dissolved in ethyl acetate, washed with brine, dried, filtered and evaporated to dryness. 0.349 g of the title compound was obtained.
¹H NMR (400 MHz, CDCl₃) δ ppm 1.83 (s, 2 H), 2.21 (s, 3 H), 2.87 (t, 2 H), 3.86 (s, 2 H), 4.15 (t, 2 H), 6.79 - 6.84 (m, 1 H), 6.89 - 6.94 (m, 2 H), 7.22 - 7.28 (m, 1 H).

### Intermediate 7: 1-(3-(Aminomethyl)phenyl)butan-1-one hydrochloride

### Step 1: 3-(2-Propyl-1,3-dioxolan-2-yl)benzonitrile

3-Butyrylbenzonitrile (3.20 mmol, 0.554 g), p-toluenesulfonic acid monohydrate (0.320 mmol, 0.061 g) and ethylene glycol (17.93 mmol, 1.113 g) were suspended in dry toluene (15 ml). The mixture was refluxed under a Dean-Stark trap for 4 hours. The cooled reaction mixture was washed with saturated NaHCO₃, water and brine. The organic layer was dried (Na₂SO₄), filtered and evaporated to dryness. The crude product was purified using silica plug filtration. 0.595 g of the title compound was obtained.
¹H NMR (400 MHz, CDCl₃) δ ppm 0.89 (t, 3 H), 1.23 - 1.39 (m, 2 H), 1.78 - 1.89 (m, 2 H), 3.70 - 3.81 (m, 2 H), 3.98 - 4.10 (m, 2 H), 7.45 (t, 1 H), 7.59 (d, 1 H), 7.69 (d, 1 H), 7.77 (s, 1 H).

### Step 2: (3-(2-Propyl-1,3-dioxolan-2-yl)phenyl)methanamine

Lithium aluminum hydride (3.95 mmol, 0.15 g) in dry tetrahydrofuran (6 ml) was stirred under nitrogen atmosphere. The mixture was cooled on an ice bath and 3-(2-propyl-1,3-dioxolan-2-yl)benzonitrile (2.74 mmol, 0.595 g) in dry tetrahydrofuran (5 ml) was added dropwise. The ice bath was removed and the mixture was stirred at room temperature for 2 hours. The reaction mixture was cooled and quenched with water and 1M NaOH. The mixture was stirred for one hour. The precipitate was filtered off, and the filtrate was evaporated to dryness. The residue was dissolved in ethyl acetate, washed with brine, dried (Na₂SO₄), filtered and evaporated. 0.544 g of the title compound was obtained.
¹H NMR (400 MHz, CDCl₃) δ ppm 0.88 (t, 3 H), 1.29 - 1.41 (m, 2 H), 1.50 (bs, 2 H), 1.84-1.92 (m, 2 H), 3.73 - 3.84 (m, 2 H), 3.89 (s, 2 H), 3.96 - 4.07 (m, 2 H), 7.21 - 7.41 (m, 4 H).

### Step 3: 1-(3-(Aminomethyl)phenyl)butan-1-one hydrochloride

(3-(2-Propyl-1,3-dioxolan-2-yl)phenyl)methanamine (2.458 mmol, 0.544 g), methanol (4 ml) and 4 M HCl (4 ml) were stirred at room temperature overnight. The solvents were evaporated. Methanol and diethyl ether were added. The formed precipitate was filtered. The precipitate was triturated with tetrahydrofuran. 0.439 g of the title compound was obtained. ¹H NMR (400 MHz, DMSO-d₆) δ ppm 0.94 (t, 3 H), 1.59 - 1.72 (m, 2 H), 3.01 (t, 2 H), 4.11 (s, 2 H), 7.57 (t, 1 H), 7.73 (d, 1 H), 7.97 (d, 1 H), 8.12 (s, 1 H), 8.35 (bs, 2 H).

### Intermediate 8: (4-Isobutoxypyridin-2-yl)methanamine hydrochloride

### Step 1: 4-Isobutoxypicolinonitrile

Sodium hydride in oil 60% (5.57 mmol, 0.223 g) and dry N,N-dimethyl formamide (7 ml) were added into a flask under nitrogen atmosphere. The mixture was cooled down to 0 °C and isobutanol (3.65 mmol, 0.271 g) was added in small portions. The ice bath was removed after 30 minutes. The resulting mixture was stirred at room temperature for 1 hour and 20 minutes. 4-Iodopicolinonitrile (3.48 mmol, 0.800 g) was added and the mixture was stirred additional 30 minutes. The reaction mixture was diluted with ethyl acetate and washed three times with water. The organic phase was dried over Na₂SO₄, filtered and evaporated. The product was purified by flash chromatography. 0.661 g of the title compound was obtained. ¹H-NMR (400 MHz, CDCl₃) δ ppm 1.04 (d, 6 H) 2.06 - 2.19 (m, 1 H) 3.81 (d, 2 H) 6.98 (dd, 1 H) 7.21 (dd, 1 H) 8.48 (dd, 1 H).

### Step 2: Tert-butyl (4-isobutoxypyridin-2-yl)methylcarbamate

4-Isobutoxypicolinonitrile (3.75 mmol, 0.660 g) was dissolved in dry methanol (40 ml) under nitrogen atmosphere. The mixture was cooled to 0 °C and di-tert-butyl dicarbonate (7.49 mmol, 1.635 g) and nickel(II) chloride hexahydrate (0.749 mmol, 0.178 g) were added. Sodium borohydride (26.20 mmol, 0.992 g) was added in small portions during 30 minutes. The mixture was allowed to warm to room temperature and stirred overnight. Diethylenetriamine (3.75 mmol, 0.386 g) was added and stirring was continued for 30 minutes. The solvent was evaporated. The precipitate was dissolved in ethyl acetate and washed twice with saturated NaHCO₃. The organic phase was dried over Na₂SO₄, filtered and evaporated. 0.613 g of the title compound was obtained. ¹H NMR (400 MHz, CDCl₃) δ ppm 1.02 (d, 6 H) 1.46 (br. s., 9 H) 2.03 - 2.15 (m, 1 H) 3.76 (d, 2 H) 4.37 (d, 2 H) 5.57 (br. s., 1 H) 6.69 (dd, 1 H) 6.77 (d, 1 H) 8.32 (d, 1 H).

### Step 3: (4-Isobutoxypyridin-2-yl)methanamine hydrochloride

Tert-butyl (4-isobutoxypyridin-2-yl)methylcarbamate (2.176 mmol, 0.610 g) was dissolved in 10 % HCl/EtOAc (2.176 mmol, 7.5 ml) under nitrogen atmosphere. The mixture was stirred at room temperature for 2 hours. The solvent was evaporated and the residue was dried. 0.598 g of the title compound was obtained.
¹H NMR (400 MHz, CD₃OD) δ ppm 1.09 (d, 6 H) 2.15 - 2.26 (m, 1 H) 4.19 (d, 2 H) 4.52 (s, 2 H) 7.55 (dd, 1 H) 7.71 (d, 1 H) 8.69 (d, 1 H).

### Intermediate 9: (2-(Neopentyloxy)pyridin-4-yl)methanamine hydrochloride

### Step 1: 2-(Neopentyloxy)isonicotinonitrile

Sodium hydride (21.65 mmol, 0.520 g) was suspended in tetrahydrofuran (20 ml) under nitrogen atmosphere at 0 °C. Neopentylalcohol (21.65 mmol, 1.909 g) was added slowly and the mixture was stirred for 20 minutes. A solution of 2-chloro-4-cyano pyridine (10.83 mmol, 1.500 g) in tetrahydrofuran (10 ml) was added dropwise. The resulting mixture was heated at 65 °C for 6h. The reaction mixture was diluted with water and extracted with ethyl acetate. The organic phase was washed with brine, dried over Na₂SO₄, filtered and evaporated. The product was purified by flash chromatography. 0.735 g of the title compound was obtained.
¹H NMR (400 MHz, CDCl₃) δ ppm 1.03 (s, 9 H) 4.00 (s, 2 H) 7.00 - 7.02 (m, 1 H) 7.04 (dd, 1 H) 8.27 (dd, 1 H).

### Step 2: Tert-butyl (2-(neopentyloxy)pyridin-4-yl)methylcarbamate

2-(Neopentyloxy)isonicotinonitrile (3.86 mmol, 0.735 g) in methanol (20 ml) was stirred at 0 °C. Nickel(II) chloride hexahydrate (0.386 mmol, 0.092 g) and di-tert-butyl dicarbonate (7.73 mmol, 1.686 g) were added and the mixture was stirred for 15 minutes. Sodium borohydride (27.00 mmol, 1.023 g) was added in small portions. The ice bath was removed and the mixture was stirred at room temperature overnight. The reaction mixture was cooled on an ice-bath. Water was added to quench the reaction. The mixture was filtered through celite and the precipitate was washed with methanol. The filtrate was concentrated. The remaining aqueous phase was extracted with ethyl acetate. Layers were separated, the organic layer was washed with water, dried over Na₂SO₄, filtered and evaporated to dryness. 0.836 g of the title compound was obtained.
¹H NMR (400 MHz, CDCl₃) δ ppm 1.02 (s, 9 H) 1.47 (bs, 9 H) 3.95 (s, 2 H) 4.28 (d, 2 H) 4.90 (bs, 1 H) 6.66 (bs, 1 H) 6.76 (d, 1 H) 8.07 (d, 1 H).

### Step 3: (2-(Neopentyloxy)pyridin-4-yl)methanamine hydrochloride

A mixture of tert-butyl (2-(neopentyloxy)pyridin-4-yl)methylcarbamate (2.84 mmol, 0.836 g) and 4 M HCl in dioxane (28 mmol, 7 ml) was stirred at room temperature for 2.5 hours. The solvent was evaporated. The mixture was cooled and diethyl ether was added. The mixture was stirred until a precipitate was formed. The precipitate was filtered, washed with heptane and dried. 0.71 g of the title compound was obtained.
¹H NMR (400 MHz, DMSO-*d*₆) δ ppm 0.99 (s, 9 H) 3.96 (s, 2 H) 4.00 - 4.06 (m, 2 H) 6.97 (dd, 1 H) 7.07 (dd, 1 H) 8.16 (d, 1 H) 8.53 (bs, 2 H).

### Intermediate 10: 4-(Aminomethyl)-2-ethylbenzonitrile

### Step 1: 4-Ethyl-4-(hydroxymethyl)benzonitrile

Sodium borohydride (7.93 mmol, 0.30 g) was added to a stirred solution of 2-ethyl-4-formylbenzonitrile (2.51 mmol, 0.40 g) in ethanol (15 ml). The mixture was stirred for 3 hours. Some of the solvent was removed by evaporation. Saturated NH₄Cl-solution (20 ml) was added. The resulting mixture was extracted twice with ethyl acetate. The combined organic layers were dried and evaporated to dryness to give 0.38 g of the title compound. ¹H NMR (400 MHz, CDCl₃) δ ppm 1.30 (t, 3 H) 1.92 (t, 1 H) 2.88 (q, 2 H) 4.75 (d, 2 H) 7.25 - 7.30 (m, 1 H) 7.33 - 7.36 (m, 1 H) 7.59 (d, 1 H).

### Step 2: 4-(Azidomethyl)-2-ethylbenzonitrile

A mixture of 2-ethyl-4-(hydroxymethyl)benzonitrile (2.376 mmol, 0.383 g), triphenylphosphine (3.56 mmol, 0.935 g) and dry tetrahydrofuran (20 ml) was cooled to 0-5 °C. Diisoproryl azodicarboxylate (3.09 mmol, 0.608 ml) was added and the mixture was stirred for 15 minutes. Diphenylphosphoryl azide (3.09 mmol, 0.669 ml) was added and the mixture was stirred at room temperature for 1 hour. The solvent was evaporated. The product was purified by flash chromatography. 0.418 g of the title compound was obtained. ¹H NMR (400 MHz, CDCl₃) δ ppm 1.31 (t, 3 H) 2.89 (q, 2 H) 4.41 (s, 2 H) 7.21 - 7.31 (m, 1 H) 7.35 - 7.42 (m, 1 H) 7.62 (d, 1 H).

### Step 3: 4-(Aminomethyl)-2-ethylbenzonitrile

4-(Azidomethyl)-2-ethylbenzonitrile (0.752 mmol, 0.200 g) was dissolved in tetrahydofuran (5 ml) and heated to 50°C. Triphenylphosphine (0.752 mmol, 0.197 g) in tetrahydrofuran (3 ml) was added in small portions during 10 minutes. The heating was continued for 30 minutes. Ethanol (4 ml) and 2M NaOH (3 ml) was added. The reaction mixture was stirred at 65 °C for an hour. The mixture was concentrated and the residue acidified with 1M HCl and washed twice with ethyl acetate. The pH of the water phase was made alkaline with 2M NaOH and extracted three times with ethyl acetate. The combined organic layers were dried, filtered and evaporated. 65 mg of the title compound was obtained.
¹H NMR (400 MHz, CDCl₃) δ ppm 1.30 (t, 3 H) 2.87 (q, 2 H) 3.93 (s, 2 H) 7.22 - 7.26 (m, 1 H) 7.31 (s, 1 H) 7.57 (d, 1 H).

### Intermediate 11: (4-(2,2,2-trifluoroethoxy)pyridin-2-yl)methanamine hydrochloride

### Step 1: 4-(2,2,2-Trifluoroethoxy)picolinonitrile

4-Iodopicolinonitrile (3.04 mmol, 0.700 g), potassium carbonate (5.48 mmol, 0.757 g) and 2,2,2-trifluoroethanol (4.26 mmol, 0.426 g) in dry N,N-dimethyl formamide (14 ml) were heated at 110 °C for 20 hours and at 120 °C for 10 hours. The solvent was removed by evaporation. The residue was dissolved in dichloromethane and washed with water. The water phase was extracted twice with dichloromethane. The combined organic phases were dried over Na₂SO₄, filtered and evaporated. The product was purified by flash chromatography. 0.469 g of the title compound was obtained.
¹H NMR (400 MHz, CDCl₃) δ ppm 4.48 (q, 2 H) 7.08 (dd, 1 H) 7.29 (dd, 1 H) 8.60 (dd, 1 H).

### Step 2: Tert-butyl (4-(2,2,2-trifluoroethoxy)pyridin-2-yl)methylcarbamate

was prepared using a procedure similar to the procedure described for tert-butyl (4-isobutoxypyridin-2-yl)methylcarbamate (in Intermediate 8), starting from 4-(2,2,2-trifluoroethoxy)picolinonitrile (2.320 mmol, 0.469 g) and di-tert-butyl dicarbonate (4.640 mmol, 1.013 g). After work up 0.591 g of the title compound was obtained.
¹H NMR (400 MHz, CDCl₃) δ ppm 1.47 (bs, 9 H) 4.35 - 4.45 (m, 4 H) 5.48 (bs, 1 H) 6.76 (dd, 1 H) 6.85 (d, 1 H) 8.42 (d, 1 H).

### Step 3: (4-(2,2,2-Trifluoroethoxy)pyridin-2-yl)methanamine hydrochloride

Tert-butyl (4-(2,2,2-trifluoroethoxy)pyridin-2-yl)methylcarbamate (1.926 mmol, 0.590 g) was dissolved in 10 % HCl/EtOAc (7 ml). The mixture was stirred at room temperature overnight. The solvent was evaporated and the residue was dried. 0.540 g of the title compound was obtained.
¹H NMR (400 MHz, CD₃OD) δ ppm 4.48 (s, 2 H) 4.98 (q, 2 H) 7.56 (dd, 1 H) 7.67 (d, 1 H) 8.76 (d, 1 H).

### Intermediate 12: 4-(Aminomethyl)-2-propylbenzonitrile

### Step 1: 4-bromo-2-propylaniline

2-Propylaniline 97% (18.49 mmol, 2.5 g) was dissolved in dry tetrahydrofuran (25 ml) under nitrogen atmosphere. The solution was cooled to 0 °C and tetrabutylammonium tribromide (18.49 mmol, 8.92 g) was added keeping the temperature below 5°C. The mixture was stirred for three hours. The mixture was concentrated, diluted with ethyl acetate and washed with saturated Na₂S₂O₅ solution, water and brine. Dried with Na₂SO₄, filtered and evaporated. The product was purified by flash chromatography. 2.92 g of the title compound was obtained.
¹H NMR (400 MHz, CDCl₃) δ ppm 1.01 (t, 3 H), 1.59 - 1.70 (m, 2 H), 2.40 - 2.47 (m, 2 H), 3.61 (bs, 2 H), 6.53-6.58 (m, 1 H), 7.10-7.14 (m, 1 H), 7.14-7.17 (m, 1 H).

### Step 2: 4-Bromo-2-propylbenzonitrile

4-Bromo-2-propylaniline (14.01 mmol, 3 g) was suspended in water. Concentrated hydrochloric acid (5.05 ml) was added and the resulting mixture was sonicated until a fine suspension was formed. The suspension was cooled to 0 °C. Sodium nitrite (15.41 mmol, 1.063 g) in water (13 ml) was added dropwise. The mixture was stirred at 0 °C for 30
minutes. The solution was neutralised with sodium hydrogencarbonate. Copper(I) cyanide (16.81 mmol, 1.506 g) and potassium cyanide (35.0 mmol, 2.281 g) were dissolved in water (15 ml) and this solution was heated to 70°C. The former solution was slowly added to the latter. After combining, the resulting solution was stirred for 30 minutes at 70°C. The reaction mixture was cooled to ambient temperature and extracted twice with toluene. The organic layers were washed with water and brine, dried, filtered and evaporated. The product was purified by flash chromatography. 2.029 g of the title compound was obtained.
¹H NMR (400 MHz, CDCl₃) δ ppm 1.00 (t, 3 H), 1.66 - 1.78 (m, 2 H), 2.76 - 2.83 (m, 2 H), 7.42 - 7.45 (m, 1 H), 7.46 - 7.49 (m, 1 H), 7.49 - 7.51 (m, 1 H).

### Step 3: 4-Formyl-2-propylbenzonitrile

4-Bromo-2-propylbenzonitrile (9.05 mmol, 2.029 g) and dry tetrahydrofuran (30 ml) were added into a dry flask under nitrogen atmosphere. The mixture was cooled to -100°C. N-Butyllithium, 1.6 M (11.77 mmol, 7.36 ml) was added in small portions. The mixture was stirred for 15 minutes.. Dry N,N-dimethylformamide (18.11 mmol, 1.40 ml) in dry tetrahydrofuran (10 ml) was added slowly. The reaction mixture was stirred for two hours while the temperature was allowed to reach r.t. The reaction mixture was poured into a NaCl-solution. The formed solution was acidified with 1M HCl and extracted three times with ether. The combined organic layers were washed with water and brine, dried, filtered and evaporated. 1.337 g of the title compound was obtained.
¹H NMR (400 MHz, CDCl₃) δ ppm 1.02 (t, 3 H), 1.72 - 1.83 (m, 2 H), 2.90 - 2.95 (m, 2 H), 7.79 - 7.81 (m, 2 H), 7.83 (s, 1 H), 10.07 (s, 1 H).

### Step 4: 4-(Hydroxymethyl)-2-propylbenzonitrile

4-Formyl-2-propylbenzonitrile (6.47 mmol, 1.4 g) was dissolved in ethanol. Sodium borohydride (19.40 mmol, 0.734 g) was added in small portions. The mixture was stirred at room temperature for 2.5 hours. Most of the ethanol was evaporated. NH₄Cl and ethyl acetate was added. The phases were separated and the water phase was extracted two more times with ethyl acetate. The combined organic layers were washed with water and brine. The organic layer was dried, filtered and evaporated. The product was purified by flash chromatography. 0.556 g of the title compound was obtained.
¹H NMR (400 MHz, CDCl₃) δ ppm 0.99 (t, 3 H), 1.66 - 1.78 (m, 2 H), 1.95 (t, 1 H), 2.79 - 2.86 (m, 2 H), 4.75 (d, 2 H), 7.26 - 7.30 (m, 1 H), 7.33 (s, 1 H), 7.59 (d, 1 H).

### Step 5: 4-(Azidomethyl)-2-propylbenzonitrile

A mixture of 4-(hydroxymethyl)-2-propylbenzonitrile (3.14 mmol, 0.55 g) and diphenylphosphoryl azide (3.77 mmol, 1.037 g) in dry toluene under nitrogen atmosphere was cooled on an ice bath. 1,8-Diazabicyclo(5,4,0)undec-7-ene (3.77 mmol, 0.573 g) was added and the resulting mixture was stirred on a thawing ice bath overnight. The mixture was diluted with toluene and washed twice with 1M HCl. The organic phase was dried, filtered and evaporated. The product was purified by flash chromatography. 0.479 g of the title compound was obtained.
¹H NMR (400 MHz, CDCl₃) δ ppm 1.00 (t, 3 H), 1.66 - 1.79 (m, 2 H), 2.79 - 2.88 (m, 2 H), 4.41 (s, 2 H), 7.21 - 7.26 (m, 1 H), 7.26 - 7.28 (m, 1 H), 7.62 (d, 1 H).

### Step 6: 4-(Aminomethyl)-2-propylbenzonitrile

4-(Azidomethyl)-2-propylbenzonitrile (2.392 mmol, 0.479 g) was dissolved in tetrahydrofuran and heated to 50 °C. Triphenylphosphine (2.392 mmol, 0.627 g) in tetrahydrofuran was added during 10 minutes. The mixture was stirred for 30 minutes. Ethanol and 2M NaOH was added. The reaction mixture was heated at 65 °C for an hour. The mixture was concentrated and the remaining aqueous solution was acidified with 1M HCl and washed twice with ethyl acetate. The aqueous phase was made alkaline with 2M NaOH and extracted three times with ethyl acetate. The combined organic layers were dried, filtered and evaporated. 0.377 g of the title compound was obtained.
¹H NMR (400 MHz, CDCl₃) δ ppm 0.99 (t, 3 H), 1.65 - 1.78 (m, 2 H), 2.76 - 2.86 (m, 2 H), 3.92 (s, 2 H), 7.22 - 7.26 (m, 1 H), 7.27 - 7.29 (m, 1 H), 7.57 (d, 1 H).

### Intermediate 13: (3-Ethoxy-5-fluorophenyl)methanamine

### Step 1: 3-Ethoxy-5-fluorobenzonitrile

A mixture of 3-fluoro-5-hydroxybenzonitrile (3.54 mmol, 0.5 g), potassium carbonate (6.01 mmol, 0.831 g), iodoethane (5.31 mmol, 0.844 g) and N,N-dimethyl formamide (4 ml) was heated 80 °C for 20 minutes using a microwave reactor. The solvent was evaporated. Water was added and the resulting mixture was extracted three times with dichloromethane. The organic phases were separated and evaporated. The residue was dried with vacuum at 40 °C. 0.544 g of the title compound was obtained.
¹H NMR (400 MHz, CDCl₃) δ ppm 1.44 (t, 3 H), 4.05 (q, 2 H), 6.81 - 6.87 (m, 1 H), 6.91 - 6.98 (m, 2 H).

### Step 2: (3-Ethoxy-5-fluorophenyl)methanamine

(3-Ethoxy-5-fluorophenyl)methanamine was prepared using a procedure similar to the procedure described for (3-(2-(methylthio)ethoxy)phenyl)methanamine (in Intermediate 6) starting from lithium aluminum hydride (8.23 mmol, 0.313 g) and 3-ethoxy-5-fluorobenzonitrile (3.29 mmol, 0.544 g). 0.462 g of the title compound was obtained.
¹H NMR (400 MHz, CDCl₃) δ ppm 1.40 (t, 3 H), 1.56 (bs, 2 H), 3.81 (s, 2 H), 4.01 (q, 2 H), 6.43-6.51 (m, 1 H), 6.57 - 6.68 (m, 2 H).

### Intermediate 14: (3-Chloro-5-methoxyphenyl)methanamine

### Step 1: 3-Chloro-5-methoxybenzonitrile

3-Chloro-5-hydroxybenzonitrile (1.302 mmol, 0.200 g), potassium carbonate (2.600 mmol, 0.360 g), N,N-dimethyl formamide (4 ml) and iodomethane (26.0 mmol, 3.700 g) were added into a flask under nitrogen atmosphere. The resulting mixture was stirred for 4 h at 70 °C. The solvent was evaporated, the residue was dissolved in dichloromethane and washed with water. The water phase was washed twice with dichloromethane. The organic phases were separated and evaporated. 0.185 g of the title compound was obtained.
¹H NMR (400 MHz, CDCl₃) δ ppm 3.84 (s, 3 H) 6.96 - 7.29 (m, 3 H).

### Step 2: (3-Chloro-5-methoxyphenyl)methanamine

Sodium borohydride (1.126 mmol, 42.6 mg) and tetrahydrofuran (1 ml) were stirred on an ice-bath, under an atmosphere of nitrogen. Trifluoroacetic acid (1.104 mmol, 126.0 mg) in tetrahydrofuran (0.5 ml) was added and the ice-bath was removed. 3-chloro-5-methoxybenzonitrile (1.104 mmol, 185.0 mg) in tetrahydrofuran was added. The mixture was stirred overnight. The reaction was quenched with water and concentrated. The remaining aqueous solution was made alkaline with 1 M NaOH and extracted three times with ethyl acetate. The combined organic layers were dried and evaporated to dryness to give 126.0 mg of the title compound.
¹H NMR (400 MHz, CDCl₃) δ ppm 2.90 (bs, 2 H) 3.71 - 3.85 (m, 5 H) 6.71 - 6.96 (br. m., 3 H).

### Intermediate 15: (3-(difluoromethoxy)-5-fluorophenyl)methanamine

### Step 1: 1-(Azidomethyl)-3-(difluoromethoxy)-5-fluorobenzene

1-(Bromomethyl)-3-(difluoromethoxy)-5-fluorobenzene (1.961 mmol, 0.500 g), sodium azide (2.940 mmol, 0.193 g) and dry ethanol (6 ml) were added into a flask under nitrogen atmosphere. The mixture was refluxed for 5 h and stirred at room temperature overnight. The solution was filtered, the solvent was evaporated and the residue was dried. 0.603 g of the title compound was obtained.
¹H NMR (400 MHz, CDCl₃) δ ppm 4.36 (s, 2 H) 6.52 (br. t., 1 H) 6.81 - 6.86 (m, 1 H) 6.87 - 6.94 (m, 2 H).

### Step 2: (3-(Difluoromethoxy)-5-fluorophenyl)methanamine

1-(Azidomethyl)-3-(difluoromethoxy)-5-fluorobenzene (0.783 mmol, 170 mg) in ethanol (24 ml) was hydrogenated over Pd/C using a continuous-flow hydrogenation reactor at 10 bar and 30 °C. The solvent was evaporated. 123 mg of the title compound was obtained.
¹H NMR (400 MHz, CD₃OD) δ ppm 1.96 (s, 2 H) 4.02 (s, 2 H) 6.76 (br. t., 2 H) 6.85 - 6.91 (m, 1 H) 7.04 - 7.09 (m, 2 H).

### Intermediate 16: (2-(Isobutylthio)pyridin-4-yl)methanamine hydrochloride

### Step 1: 2-(Isobutylthio)isonicotinonitrile

2-Methylpropan-1-thiol (72.0 mmol, 6.45 g) and sodium methoxide (54.0 mmol, 2.9 g) were dissolved in ice cold tetrahydrofuran and stirred at 0 °C for 10 minutes. A solution of 2-chloroisonicotinonitrile (36.0 mmol, 5 g) in tetrahydrofuran was added dropwise and heated at 60 °C overnight. The reaction mixture was poured into ice water and extracted with EtOAc. The layers were separated and the aqueous layer was washed with brine, dried (Na₂SO₄), filtered and concentrated. The product was purified by column chromatography. 2.1 g of the title compound was obtained.
¹H-NMR (400 MHz, CDCl₃): δ 8.54 (d, 1H), 7.37 (d, 1H), 7.12 (s, 1H), 3.1 (d, 2H), 1.97-2.0 (m, 1H), 1.05 (d, 6H).

### Step 2: Tert-butyl ((2-(isobutylthio)pyridin-4-yl)methyl)carbamate

2-(Isobutylthio)isonicotinonitrile (11.0 mmol, 2.1 g), nickel chloride (1.10 mmol, 0.25 g) and boc-anhydride (22.0 mmol, 5.6 ml) were dissolved in methanol and stirred at room temperature for 10 minutes. Sodium borohydride (140 mmol, 2.7 g) was added in small portions. The mixture was stirred overnight at room temperature. The reaction mixture was concentrated, quenched with water and extracted with EtOAc. The organic phase was washed with water, dried (Na₂SO₄), filtered and concentrated under reduced pressure. The crude product was purified by column chromatography. 1.2 g of the title compound was obtained.
¹H-NMR (400 MHz; CDCl₃): δ 8.34 (d, 1H), 7.07 (s, 1H), 6.86 (d, 1H), 4.81 (s, 1H), 4.25 (s, 2H), 3.09 (d, 2H), 1.89-1.91 (m, 1H), 1.47 (s, 9H), 1.03 (d, 6H).

### Step 3: (2-(Isobutylthio)pyridin-4-yl)methanamine hydrochloride

Tert-butyl ((2-(isobutylthio)pyridin-4-yl)methyl)carbamate (3.4 mmol, 1.0 g) and 4N HCl/dioxane (10 ml) was stirred at r.t. for 3 h. The reaction mixture was concentrated under reduced pressure, triturated with ether and pentane repeatedly until a precipitate was formed. It was sonicated with pentane and dried under vacuum. 0.90 g of the title compound was obtained.
¹H-NMR (400 MHz, DMSO-*d₆*): δ 8.61 (s, 2H), 8.44 (d, 1H), 7.46 (s, 1H), 7.23 (d, 1H), 4.21(s, 1H), 4.03 (s, 2H), 3.09 (d, 2H), 1.90-1.92 (m, 1H), 0.98 (d, 6H).

### Intermediate 17: 5-(aminomethyl)-2-chloro-N-isopropylaniline

### Step 1: 4-Chloro-3-(isopropylamino)benzamide

3-Amino-4-chlorobenzamide (5.86 mmol, 1.000 g), acetic acid, glacial (35.2 mmol, 2.112 g), acetone 99.9 % (11.14 mmol, 0.819 ml) and 1,2-dichloroethane (30 ml) were stirred under nitrogen atmosphere. Sodium triacetoxy borohydride (16.41 mmol, 3.480 g) was added and the resulting mixture was stirred at room temperature for two days. The reaction mixture was neutralised by adding saturated NaHCO₃ solution and extracted three times with ethyl acetate. The combined organic phases were dried, filtered and evaporated. The product was purified by flash chromatography. 1.144 g of the title compound was obtained.
¹H NMR (400 MHz, CD₃OD) δ ppm 1.26 (d, 6 H) 3.72 - 3.84 (m, 1 H) 7.05 - 7.09 (m, 1 H) 7.19 - 7.24 (m, 1 H) 7.27-7.31 (m, 1 H).

### Step 2: 5-(Aminomethyl)-2-chloro-N-isopropylaniline

Lithium aluminum hydride (10.76 mmol, 0.408 g) and tetrahydrofuran (20 ml) were stirred under nitrogen atmosphere at 0 °C in an ice bath. A solution of 4-chloro-3-(isopropylamino)benzamide (5.38 mmol, 1.144 g) in tetrahydrofuran (10 ml) was added dropwise. The ice bath was removed after 5 minutes and the resulting mixture was refluxed for 4 hours. The mixture was cooled on an ice bath, and the reaction was quenched by adding water dropwise and stirring vigorously for 15 minutes. The solids were filtered off and washed with dichloromethane. The filtrate was neutralized by adding 1M HCl and stirred for 10 minutes on a cold bath. The water phase was separated and evaporated. The product was purified by reverse phase chromatography. 0.515 g of the title compound was obtained.
¹H NMR (400 MHz, CD₃OD) δ ppm 1.27 (d, 6 H) 3.70 - 3.81 (m, 1 H) 4.03 (s, 2 H) 6.63 - 6.68 (m, 1 H) 6.78 - 6.85 (m, 1 H) 7.25 - 7.30 (m, 1 H).

### Intermediate 18: (4-(trifluoromethyl)pyridin-2-yl)methanamine hydrochloride.

### Step 1: Tert-butyl (4-(trifluoromethyl)pyridin-2-yl)methylcarbamate

was prepared as described for tert-butyl (4-isobutoxypyridin-2-yl)methylcarbamate, (in Intermediate 8) starting from 4-(trifluoromethyl)picolinonitrile (1.743 mmol, 0.300 g) and di-tert-butyl dicarbonate (3.490 mmol, 0.761 g). 0.419 g of the title compound was obtained. ¹H NMR (400 MHz, CDCl₃) δ ppm 1.47 (br. s., 9 H) 4.53 (d, 2 H) 5.51 (br. s., 1 H) 7.41 (d, 1 H) 7.50 (s, 1 H) 8.72 (d, 1 H).

### Step 2: (4-(Trifluoromethyl)pyridin-2-yl)methanamine hydrochloride

Tert-butyl (4-(trifluoromethyl)pyridin-2-yl)methylcarbamate (1.517 mmol, 0.419 g) was dissolved in 10 % HCl/EtOAc (1.517 mmol, 3 ml) under nitrogen atmosphere. The mixture was stirred at room temperature overnight. The solvent was evaporated and the residue was dried. 0.342 g of the title compound was obtained.
¹H NMR (400 MHz, CD₃OD) δ ppm 4.43 (s, 2 H) 7.72 (d, 1 H) 7.84 (s, 1 H) 8.89 (d, 1 H).

### Intermediate 19: (5-(Isopentyloxy)pyridin-3-yl)methanamine hydrochloride

### Step 1: 3-Bromo-5-(isopentyloxy)pyridine

5-Bromopyridin-3-ol (4.60 mmol, 0.800 g) was dissolved in acetone (20 ml). Potassium carbonate (5.98 mmol, 0.826 g) and 1-bromo-3-methylbutane (5.98 mmol, 0.903 g) were added and the resulting mixture was refluxed for 5 hours and 100 °C for 3 hours under microwave radiation. The precipitate was filtered off. The filtrate was evaporated to dryness. The resulting residue was dissolved in ethyl acetate and washed by water. The organic phase was dried over Na₂SO₄, filtered and evaporated. 0.877 g of the title compound was obtained. ¹H NMR (400 MHz, CDCl₃) δ ppm 0.97 (d, 6 H) 1.69 (q, 2 H) 1.77 - 1.89 (m, 1 H) 4.02 (t, 2 H) 7.35 (dd, 1 H) 8.22 (d, 1 H) 8.26 (d, 1 H).

### Step 2: 5-(Isopentyloxy)nicotinonitrile

3-Bromo-5-(isopentyloxy)pyridine (3.59 mmol, 0.877 g), copper(I) cyanide (3.59 mmol, 0.322 g) and N,N-dimethyl formamide (10 ml) were heated at 190 °C for 1 hour and 50 minutes under microwave radiation. The reaction mixture was added to a 15 % ammonia solution (8 ml) and stirred vigorously. The resulting mixture was extracted three times with dichloromethane. The organic phases were dried over Na₂SO₄, filtered and evaporated to dryness. 0.641 g of the title compound was obtained.
¹H NMR (400 MHz, CDCl₃) δ ppm 0.98 (d, 6 H) 1.68 - 1.76 (m, 2 H) 1.79 - 1.90 (m, 1 H) 4.06 (t, 2 H) 7.39 (dd, 1 H) 8.46 (d, 1 H) 8.49 (d, 1 H).

### Step 3: Tert-butyl (5-(isopentyloxy)pyridin-3-yl)methylcarbamate

5-(Isopentyloxy)nicotinonitrile (3.37 mmol, 0.641 g) was dissolved in dry methanol (20 ml) under nitrogen atmosphere. The mixture was cooled to 0 °C and di-tert-butyl dicarbonate (6.74 mmol, 1.471 g) and nickel(II) chloride hexahydrate (0.337 mmol, 0.080 g) were added. Sodium borohydride (23.59 mmol, 0.892 g) was added in small portions during 30 minutes. The mixture was allowed to warm to room temperature and stirred for 4 hours. Diethylenetriamine (3.37 mmol, 0.348 g) was added and stirring was continued for 1 hour. The solvent was evaporated. The precipitate was dissolved in dichloromethane and washed twice with saturated NaHCO₃. The organic phase was dried over Na₂SO₄, filtered and evaporated. The product was purified by flash chromatography. 0.187 g of the title compound was obtained.
¹H NMR (400 MHz, CDCl₃) δ ppm 0.97 (d, 6 H) 1.46 (br. s., 9 H) 1.69 (q, 2 H) 1.77 - 1.90 (m, 1 H) 4.02 (t, 2 H) 4.31 (d, 2 H) 4.92 (br. s., 1 H) 7.14 (s, 1 H) 8.11 (s, 1 H) 8.20 (s, 1 H).

### Step 4: (5-(Isopentyloxy)pyridin-3-yl)methanamine hydrochloride

Tert-butyl (5-(isopentyloxy)pyridin-3-yl)methylcarbamate (0.635 mmol, 0.187 g) and dry methanol (20 ml) were added into a flask under nitrogen atmosphere. The mixture was cooled to 0 °C and thionyl chloride (5.72 mmol, 0.680 g) was added dropwise. The resulting mixture was stirred at room temperature for 4 hours. Most of the solvent was evaporated and small amount of diethyl ether was added. The mixture was stirred at room temperature until a precipitate was formed. The precipitate was filtered, washed with cold diethyl ether and dried. 0.127 g of the title compound was obtained.
¹H NMR (400 MHz, D₂O) d ppm 0.95 (d, 6 H) 1.73 (q, 2 H) 1.76 - 1.88 (m, 1 H) 4.27 (t, 2 H) 4.36 (s, 2 H) 7.98 (s, 1 H) 8.39 (s, 1 H) 8.45 (d, 1 H).

### Intermediate 20: (4-(Isobutylthio)pyridin-2-yl)methanamine hydrochloride

### Step 1: 4-(Isobutylthio)picolinonitrile

4-Iodopicolinonitrile (0.870 mmol, 0.200 g), potassium carbonate (1.565 mmol, 0.216 g), dry N,N-dimethyl formamide (3 ml) and 2-methylpropane-1-thiol (1.217 mmol, 0.110 g) were added into a microwave vial. The resulting mixture was heated by microwaves at 80 °C for 80 minutes. The solvent was evaporated. The residue was dissolved in dichloromethane and washed with water. The water phase was extracted twice with dichloromethane. The combined organic phases were evaporated to dryness. 0.171 g of the title compound was obtained.
¹H NMR (400 MHz, CDCl₃) δ ppm 1.09 (d, 6 H) 1.92 - 2.04 (m, 1 H) 2.89 (d, 2 H) 7.28 (dd, 1 H) 7.46 (dd, 1 H) 8.43 (dd, 1 H).

### Step 2: Tert-butyl (4-(isobutylthio)pyridin-2-yl)methylcarbamate

4-(Isobutylthio)picolinonitrile (0.869 mmol, 0.167 g) was dissolved in dry methanol (10 ml) under nitrogen atmosphere. The mixture was cooled to 0 °C and di-tert-butyl dicarbonate (1.737 mmol, 0.379 g) and nickel(II) chloride hexahydrate (0.174 mmol, 0.041 g) were added. Sodium borohydride (6.080 mmol, 0.230 g) was added in small amounts during 30 minutes. The mixture was allowed to warm to room temperature and stirred overnight. Diethylenetriamine (0.869 mmol, 0.090 g) was added and stirring was continued for 30 minutes. The solvent was evaporated. The precipitate was dissolved in ethyl acetate and washed twice with saturated NaHCO₃. The organic phase was dried over Na₂SO₄, filtered and evaporated. 0.168 g of the title compound was obtained.
¹H NMR (400 MHz, CDCl₃) δ ppm 1.07 (d, 6 H) 1.47 (br. s., 9 H) 1.89 - 2.01 (m, 1 H) 2.84 (d, 2 H) 4.37 (d, 2 H) 5.47 (br. s., 1 H) 6.99 (dd, 1 H) 7.08 (d, 1 H) 8.29 (d, 1 H).

### Step 3: (4-(Isobutylthio)pyridin-2-yl)methanamine hydrochloride

Tert-butyl(4-isobutylthio)pyridin-2-yl)methylcarbamate (0.567 mmol, 0.168 g) was dissolved in 10 % HCl/EtOAc (0.567 mmol, 2.0 ml) under nitrogen atmosphere. The mixture was stirred at room temperature for 2 hours. The solvent was evaporated and the residue was dried. 0.158 g of the title compound was obtained.
¹H NMR (400 MHz, CH₃OD) d ppm 1.14 (d, 6 H) 1.97 - 2.13 (m, 1 H) 3.19 (d, 2 H) 4.49 (s, 2 H) 7.82 (dd, 1 H) 7.97 (d, 1 H) 8.52 (d, 1 H).

### Intermediate 21: (3-(Pentafluorothio)phenyl)methanamine

Lithium aluminumhydride (2.62 mmol, 0.099 g) and dry diethyl ether (5 ml) were added into a flask under nitrogen atmosphere at 0 °C in an ice bath. 3-(pentafluorothio)benzonitrile (2.182 mmol, 0.500 g) was added. The resulting mixture was refluxed for two hours. The reaction mixture was cooled to 0 °C and the reaction was quenched by adding 1M NaOH (2 ml). The solvent was evaporated and methanol was added. The resulting precipitate was washed with methanol and discarded. The filtrate was dried over Na₂SO₄, filtered and evaporated. 0.547 g of the title compound was obtained.
¹H NMR (400 MHz, CDCl₃) δ ppm 3.92 (s, 2 H) 4.19 (br. s., 2 H) 7.42 - 7.51 (m, 2 H) 7.63 - 7.68 (m, 1 H) 7.70 - 7.74 (m, 1 H).

### Intermediate 22: (2-(3,3,3-Trifluoropropoxy)pyridin-4-yl)methanamine hydrochloride

### Step 1: 2-(3,3,3-Trifluoropropoxy)isonicotinonitrile

A suspension of sodium hydride (28.90 mmol, 0.693 g) and dry tetrahydrofuran (25 ml) was stirred under nitrogen atmosphere at 0 °C on an ice bath. 3,3,3-Trifluoro-1-propanol (28.90 mmol, 3.290 g) was added slowly and the mixture was stirred for 20 minutes. A solution of 2-chloro-4-cyano pyridine (14.43 mmol, 2.000 g) in tetrahydrofuran (10 ml) was added dropwise under cold conditions. The resulting mixture was heated at 65 °C for 4 hours. The reaction mixture was diluted with water and extracted with ethyl acetate. The organic phase was washed with brine, dried over Na₂SO₄, filtered and evaporated. The product was purified by flash chromatography. 0.950 g of the title compound was obtained.
¹H NMR (400 MHz, DMSO-*d*₆) δ ppm 2.73 - 2.90 (m, 2 H) 4.54 (t, 2 H) 7.40 - 7.43 (m, 1 H) 7.45 (dd, 1 H) 8.42 (dd, 1 H).

### Step 2: Tert-butyl (2-(3,3,3-trifluoropropoxy)pyridin-4-yl)methylcarbamate

Tert-butyl (2-(3,3,3-trifluoropropoxy)pyridin-4-yl)methylcarbamate was prepared using a similar procedure as described for tert-butyl (2-(neopentyloxy)pyridin-4-yl)methylcarbamate (in Intermediate 9), starting from 2-(3,3,3-trifluoropropoxy)isonicotinonitrile (4.39 mmol, 0.950 g) and di-tert-butyl dicarbonate (8.79 mmol, 1.918 g). After work up 1.082 g of the title compound was obtained.
¹H NMR (400 MHz, CDCl₃) δ ppm 1.46 (s, 9 H) 2.53 - 2.67 (m, 2 H) 4.28 (d, 2 H) 4.54 (t, 2 H) 4.92 (br. s., 1 H) 6.65 (s, 1 H) 6.81 (d, 1 H) 8.07 (d, 1 H).

### Step 3: (2-(3,3,3-Trifluoropropoxy)pyridin-4-yl)methanamine hydrochloride

Tert-butyl (2-(3,3,3-trifluoropropoxy)pyridin-4-yl)methylcarbamate (3.38 mmol, 1.082 g) in 4 M HCl/dioxane (3.38 mmol, 8 ml) was stirred for 3 hours and evaporated to dryness. 0.93 g of the title compound was obtained.
¹H NMR (400 MHz, DMSO-*d*₆) δ ppm 2.72 - 2.86 (m, 2 H) 3.99 - 4.08 (m, 2 H) 4.50 (t, 2 H) 6.98 (dd, 1 H) 7.14 (dd, 1 H) 8.20 (dd, 1 H) 8.62 (br. s., 2 H).

### Intermediate 23: 4-(Aminomethyl)-2-isopropylbenzonitrile hydrochloride

### Step 1: 4-Bromo-2-isopropylbenzonitrile

4-Bromo-2-isopropylaniline (18.92 mmol, 4.05 g) was suspended in water. Hydrogen chloride (224 mmol, 8.16 g) was added and the resulting mixture was sonicated until it formed a fine suspension. The suspension was cooled to 0 °C with an ice bath. Sodium nitrite (21.17 mmol, 1.460 g) in water was added dropwise. This solution was stirred at 0 °C for an hour. The solution was neutralized with solid sodium hydrogencarbonate. Copper(I) cyanide (22.89 mmol, 2.050 g) and potassium cyanide (47.5 mmol, 3.10 g) were dissolved in water and this solution was heated to 70°C. The former solution was slowly added to the latter solution. After combining, the solution was stirred for 2.5 hours at 70°C. The reaction mixture was cooled to ambient temperature and extracted twice with toluene. The organics were pooled, washed with water and brine, dried, filtered and evaporated. The product was purified with flash chromatography. 2.79 g of the title compound was obtained.
¹H NMR (400 MHz, CDCl₃) δ ppm 1.32 (d, 6 H), 3.30 - 3.41 (m, 1 H), 7.42 - 7.45 (m, 1 H), 7.46 - 7.49 (m, 1 H), 7.54 (d, 1 H).

### Step 2: 4-Formyl-2-isopropylbenzonitrile

4-Bromo-2-isopropylbenzonitrile (12.45 mmol, 2.79 g) and dry tetrahydrofuran were added into a dry flask under nitrogen atmosphere. The mixture was cooled to -100°C. N-Butyllithium, 1.6 M (15.20 mmol, 9.5 ml) was added in small portions and stirred for 15 minutes after the last addition. A dry and cold solution of N,N-dimethylformamide (32.3 mmol, 2.360 g) in dry and cold tetrahydrofuran was slowly added. The reaction mixture was left to react in a slowly warming bath for an hour after which the bath was removed and the mixture was left to warm to ambient temperature. The reaction mixture was poured to a half saturated NaCl-solution. The solution was acidified with 2M HCl and extracted three times with ether. The combined organics were washed with water and brine, dried, filtered and evaporated. The product was purified with flash chromatography. 1.01 g of the title compound was obtained.
¹H NMR (400 MHz, DMSO-*d*₆) δ ppm 1.32 (d, 6 H), 3.28 - 3.38 (m, 1H), 7.89 (dd, 1 H), 8.02 (d, 1 H), 8.05 - 8.07 (m, 1 H), 10.10 (s, 1 H).

### Step 3: 4-((Hydroxyimino)methyl)-2-isopropylbenzonitrile

4-Formyl-2-isopropylbenzonitrile (1.126 mmol, 0.195 g) was suspended in ethanol. Hydroxylamine hydrochloride (1.238 mmol, 0.086 g) dissolved in water was added. Anhydrous sodium acetate (1.238 mmol, 0.102 g) was added and the resulting mixture was stirred at 65 °C for 2.5 hours. Water was added and the water phase was extracted three times with dichloromethane. The combined organic layers were washed with water and brine, dried, filtered and evaporated. 0.202 g of the title compound was obtained.
¹H NMR (400 MHz, DMSO-*d*₆) δ ppm 1.28 (d, 6 H), 3.20 - 3.28 (m, 1 H), 7.60 (dd, 1 H), 7.71 - 7.74 (m, 1 H), 7.79 (d, 1 H), 8.23 (s, 1 H), 11.67 (s, 1 H).

### Step 4: 4-(Aminomethyl)-2-isopropylbenzonitrile hydrochloride

4-((Hydroxyimino)methyl)-2-isopropylbenzonitrile (1.063 mmol, 0.2 g) was dissolved in acetic acid (4 ml). Zinc (3.67 mmol, 0.24 g) was added and the resulting mixture was stirred at 65 °C for one hour. More zinc (3.67 mmol, 0.24 g) was added into the hot reaction mixture in small poriotns. Stirring continued at 65 °C for another 1.5 hours. Ethanol was added to the cooled reaction mixture and the mixture was filtered through Celite. Solvents were removed by evaporation. Ethanol (2 ml) and hydrogen chloride, 1M in ether (1.300 mmol, 1.560 mg) were added. The resulting mixture was stirred for 30 minutes at room temperature. The solvents were evaporated and ether was added. The precipitated product was filtered off, flushed with ether and dried. 176 mg of the title compound was obtained. ¹H NMR (400 MHz, DMSO-*d*₆) δ ppm 1.29 (d, 6 H), 3.19 - 3.28 (m, 1 H), 4.11 (s, 2 H), 7.42 - 7.49 (m, 1 H), 7.66 (s, 1 H), 7.84 (d, 1 H), 7.92 - 8.24 (m, 2 H).

### Preparation of the compounds of invention

### EXAMPLE 1: (S)-1-(4-Fluorophenylsulfonyl)-N-(3-(2-(methylthio)ethoxy)benzyl) pyrrolidine-2-carboxamide

(S)-1-(4-Fluorophenylsulfonyl)pyrrolidine-2-carboxylic acid (0.253 mmol, 0.069 g), (3-(2-(methylthio)ethoxy)phenyl)methanamine (0.253 mmol, 0.05 g), 1-hydroxybenzotriazole (0.279 mmol, 0.038 g), 1-(3-dimethylaminopropyl)-3-ethylcarbodiimide hydrochloride (0.279 mmol, 0.053 g), N,N-diisopropylethylamine (0.279 mmol, 0.036 g) were dissolved in dichloromethane (5 ml). The reaction mixture was stirred at room temperature for 5 hours. 5 ml of dichloromethane was added and the mixture was washed with 1M HCl and 1M Na₂CO₃. The organic phase was dried (Na₂SO₄), filtered and evaporated to dryness. The product was purified by flash chromatography. 0.037 g of the title compound was obtained. ¹H NMR (400 MHz, CDCl₃) δ ppm 1.62 - 1.87 (m, 3 H), 2.16 - 2.30 (m, 4 H), 2.89 (t, 2 H), 3.12 - 3.22 (m, 1 H), 3.52 - 3.62 (m, 1 H), 4.13 (dd, 1 H), 4.15 - 4.23 (m, 2 H), 4.40 - 4.59 (m, 2 H), 6.80 - 6.93 (m, 3 H), 7.14 - 7.21 (m, 1 H), 7.21 - 7.30 (m, 3 H), 7.83 - 7.92 (m, 2 H).

Compounds of example 2 to 34 were prepared using methods similar or analogous to the method described for example 1. In those cases where the products were expected to be basic, the washing with diluted acid during work-up was omitted.

### EXAMPLE 2: (S)-N-(3-bromobenzyl)-1-(phenylsulfonyl)pyrrolidine-2-carboxamide

was prepared starting from (S)-1-(phenylsulfonyl)pyrrolidine-2-carboxylic acid (2 mmol, 0.511 g) and 3-bromobenzylamine hydrochloride (2.0 mmol, 0.445 g). The product was purified by flash chromatography. 0.697 g of the title compound was obtained.
¹H NMR (400 MHz, CDCl₃) δ ppm 1.53 - 1.69 (m, 2 H), 1.69 - 1.83 (m, 1 H), 2.19 - 2.28 (m, 1 H), 3.17 - 3.26 (m, 1 H), 3.53 - 3.62 (m, 1 H), 4.09 - 4.20 (m, 1 H), 4.49 (d, 2 H), 7.18 - 7.35 (m, 3 H), 7.39 - 7.44 (m, 1 H), 7.46 (s, 1 H), 7.54 - 7.62 (m, 2 H), 7.63 - 7.71 (m, 1 H), 7.86 (d, 2 H).

### EXAMPLE 3: (S)-N-(3-butyrylbenzyl)-1-(4-fluorophenylsulfonyl)pyrrolidine-2-carboxamide

was prepared starting from (S)-1-(4-fluorophenylsulfonyl)pyrrolidine-2-carboxylic acid (0.200 mmol, 0.055 g) and 1-(3-(aminomethyl)phenyl)butan-1-one hydrochloride (0.240 mmol, 0.051 g). The product was purified by flash chromatography. 0.057 g of the title compound was obtained.
¹H NMR (400 MHz, CDCl₃) δ ppm 1.00 (t, 3 H) 1.60 - 1.88 (m, 5 H) 2.20 - 2.29 (m, 1 H) 2.93 - 3.00 (m, 2 H) 3.09 - 3.23 (m, 1 H) 3.52 - 3.66 (m, 1 H) 4.13 (dd, 1 H) 4.46 - 4.69 (m, 2 H) 7.21 - 7.28 (m, 2 H) 7.32 (t, 1 H) 7.40 - 7.48 (m, 1 H) 7.48 - 7.54 (m, 1 H) 7.83 - 7.93 (m, 4 H).

### EXAMPLE 4: (2S,4R)-4-Fluoro-1-(4-fluorophenylsulfonyl)-N-((2-(neopentyloxy) pyridin-4-yl)methyl)pyrrolidine-2-carboxamide

was prepared starting from (2S,4R)-4-fluoro-1-((4-fluorophenyl)sulfonyl)pyrrolidine-2-carboxylic acid (0.343 mmol, 0.100 g) and (2-(neopentyloxy)pyridin-4-yl)methanamine hydrochloride (0.343 mmol, 0.079 g). The product was purified using preparative HPLC. 0.048 g of the title compound was obtained.
¹H NMR (400 MHz, CDCl₃) δ ppm 1.02 (bs, 9 H) 2.18 - 2.37 (m, 1 H) 2.43 - 2.58 (m, 1 H) 3.53 - 3.70 (m, 1 H) 3.83 - 3.92 (m, 1 H) 3.94 (bs, 2 H) 4.28 (t, 1 H) 4.37 - 4.55 (m, 2 H) 4.95 - 5.13 (m, 1 H) 6.71 - 6.74 (m, 1 H) 6.80 - 6.85 (m, 1 H) 7.18 - 7.25 (m, 2 H) 7.40 (bs, 1 H) 7.83 - 7.91 (m, 2 H) 8.10 (dd, 1 H).

### EXAMPLE 5: (S)-N-(4-cyano-3-ethylbenzyl)-1-(4-fluorophenylsulfonyl)pyrrolidine-2-carboxamide

was prepared starting from (S)-1-(4-fluorophenylsulfonyl)pyrrolidine-2-carboxylic acid (0.369 mmol, 0.101 g) and 4-(aminomethyl)-2-ethylbenzonitrile (0.406 mmol, 0.065 g). The product was purified by flash chromatography. 0.137 g of the title compound was obtained. ¹H NMR (400 MHz, CDCl₃) δ ppm 1.30 (t, 3 H) 1.62 - 1.86 (m, 3 H) 2.19 - 2.28 (m, 1 H) 2.87 (q, 2 H) 3.11 - 3.20 (m, 1 H) 3.56 - 3.63 (m, 1 H) 4.08 - 4.15 (m, 1 H) 4.41 - 4.68 (m, 2 H) 7.20 - 7.37 (m, 5 H) 7.58 (d, 1 H) 7.84 - 7.91 (m, 2 H).

### EXAMPLE 6: (S)-1-(Phenylsulfonyl)-N-(3-propoxybenzyl)pyrrolidine-2-carboxamide

was prepared starting from (S)-1-(phenylsulfonyl)pyrrolidine-2-carboxylic acid (2.476 mmol, 0.632 g) and (3-propoxyphenyl)methanamine (2.72 mmol, 0.45 g). The product was purified by flash chromatography. 0.907 g of the title compound was obtained.
¹H NMR (400 MHz, CDCl₃) δ ppm 1.03 (t, 3 H), 1.53 - 1.66 (m, 2 H), 1.68 - 1.85 (m, 3 H), 2.17 - 2.27 (m, 1 H), 3.14 - 3.24 (m, 1 H), 3.50 - 3.59 (m, 1 H), 3.88 - 3.99 (m, 2 H), 4.13-4.19 (m, 1 H), 4.48 (d, 2 H), 6.78 - 6.91 (m, 3 H), 7.18 - 7.28 (m, 2 H), 7.52 - 7.69 (m, 3 H), 7.81 - 7.88 (m, 2 H).

### EXAMPLE 7: (S)-1-(4-Fluorophenylsulfonyl)-N-((4-(2,2,2-trifluoroethoxy)pyridin-2-yl)methyl)pyrrolidine-2-carboxamide

was prepared starting from (S)-1-(4-fluorophenylsulfonyl)pyrrolidine-2-carboxylic acid (0.293 mmol, 0.080 g) and (4-(2,2,2-trifluoroethoxy)pyridin-2-yl)methanamine hydrochloride (0.293 mmol, 0.071 g). The product was purified using preparative HPLC. 0.023 g of the title compound was obtained.
¹H NMR (400 MHz, CDCl₃) δ ppm 1.62 - 1.93 (m, 3 H) 2.14 - 2.23 (m, 1 H) 3.13 - 3.23 (m, 1 H) 3.59 - 3.67 (m, 1 H) 4.16 (dd, 1 H) 4.40 - 4.51 (m, 3 H) 4.85 (dd, 1 H) 6.80 (dd, 1 H) 7.03 (d, 1 H) 7.22 - 7.30 (m, 2 H) 7.58 (t, 1 H) 7.86 - 7.93 (m, 2 H) 8.41 (d, 1 H).

### EXAMPLE 8: (2S,4R)-4-Fluoro-1-(4-fluorophenylsulfonyl)-N-(4-(trifluoromethyl) benzyl)pyrrolidine-2-carboxamide

was prepared starting from (2S,4R)-4-fluoro-1-((4-fluorophenyl)sulfonyl)pyrrolidine-2-carboxylic acid (4.81 mmol, 1.4 g) and 4-(trifluoromethyl)benzylamin (6 mmol, 1.051 g). The product was purified by flash chromatography. 1.545 g of the title compound was obtained. ¹H NMR (400 MHz, CDCl₃) δ ppm 2.17 - 2.38 (m, 1 H), 2.43 - 2.59 (m, 1 H), 3.51 - 3.70 (m, 1 H), 3.83 - 3.97 (m, 1 H), 4.27 (t, 1 H), 4.47 - 4.68 (m, 2 H), 4.94 - 5.14 (m, 1 H), 7.18 - 7.26 (m, 2 H), 7.29 - 7.37 (m, 1 H), 7.47 (d, 2 H), 7.62 (d, 2 H), 7.83 - 7.92 (m, 2 H).

### EXAMPLE 9: (S)-N-(4-bromo-2-fluorobenzyl)-1-(4-fluorophenylsulfonyl)pyrrolidine-2-carboxamide

was prepared starting from (S)-1-(4-fluorophenylsulfonyl)pyrrolidine-2-carboxylic acid (0.293 mmol, 0.080 g) and 4-bromo-2-fluorobenzylamine hydrochloride (0.293 mmol, 0.070 g). The product was purified by flash chromatography. 0.088 g of the title compound was obtained.
¹H NMR (400 MHz, CDCl₃) δ ppm 1.56 - 1.83 (m, 3 H) 2.15 - 2.25 (m, 1 H) 3.10 - 3.21 (m, 1 H) 3.52-3.61 (m, 1 H) 4.07- 4.12 (m, 1 H) 4.42 - 4.57 (m, 2 H) 7.20 - 7.29 (m, 6 H) 7.83 - 7.89 (m, 2 H).

### EXAMPLE 10: (S)-1-(3,5-Difluorophenylsulfonyl)-N-((2-(3,3,3-trifluoropropoxy) pyridin-4-yl)methyl)pyrrolidine-2-carboxamide

was prepared starting from (S)-1-(3,5-difluorophenylsulfonyl)pyrrolidine-2-carboxylic acid (0.343 mmol, 0.100 g) and (2-(3,3,3-trifluoropropoxy)pyridin-4-yl)methanamine hydrochloride (0.343 mmol, 0.088 g). The product was purified by flash chromatography. 0.073 g of the title compound was obtained.
¹H NMR (400 MHz, CDCl₃) δ ppm 1.65 - 1.92 (m, 3 H) 2.25 - 2.34 (m, 1 H) 2.54 - 2.68 (m, 2 H) 3.16 - 3.26 (m, 1 H) 3.59 - 3.67 (m, 1 H) 4.15 (dd, 1 H) 4.39 - 4.57 (m, 4 H) 6.68 (d, 1 H) 6.85 (dd, 1 H) 7.09 - 7.21 (m, 2 H) 7.37 - 7.44 (m, 2 H) 8.10 (d, 1 H).

### EXAMPLE 11: (S)-N-(4-cyano-3-propylbenzyl)-1-(phenylsulfonyl)pyrrolidine-2-carboxamide

was prepared starting from (S)-1-(phenylsulfonyl)pyrrolidine-2-carboxylic acid (0.402 mmol, 0.103 g) and 4-(aminomethyl)-2-propylbenzonitrile (0.402 mmol, 0.07 g). The product was purified by preparative HPLC. 122 mg of the title compound was obtained.
¹H NMR (400 MHz, CDCl₃) δ ppm 0.96 (t, 3 H), 1.56 - 1.85 (m, 5 H), 2.10 - 2.21 (m, 1 H), 2.73 - 2.82 (m, 2 H), 3.14 - 3.24 (m, 1 H), 3.56 - 3.65 (m, 1 H), 4.16 (dd, 1 H), 4.42 - 4.51 (m, 1 H)f, 4.54 - 4.64 (m, 1 H), 7.21 - 7.26 (m, 1 H), 7.28 - 7.31 (m, 1 H), 7.50 - 7.62 (m, 4 H), 7.64 - 7.70 (m, 1 H), 7.81 - 7.87 (m, 2 H).

### EXAMPLE 12: (2S,4R)-N-(4-bromo-2-fluorobenzyl)-4-fluoro-1-(4-fluorophenyl-sulfonyl)pyrrolidine-2-carboxamide

was prepared starting from (2S,4R)-4-fluoro-1-(4-fluorophenylsulfonyl)pyrrolidine-2-carboxylic acid (0.275 mmol, 0.080 g) and 4-bromo-2-fluorobenzylamine hydrochloride (0.275 mmol, 0.066 g). The product was purified by flash chromatography. 0.085 g of the title compound was obtained.
¹H NMR (400 MHz, CDCl₃) δ ppm 2.14 - 2.33 (m, 1 H) 2.39 - 2.54 (m, 1 H) 3.50 - 3.67 (m, 1 H) 3.81 - 3.94 (m, 1 H) 4.23 (t, 1 H) 4.41 - 4.57 (m, 2 H) 4.92 - 5.12 (m, 1 H) 7.16 - 7.33 (m, 6 H) 7.82 - 7.88 (m, 2 H).

### EXAMPLE 13: (S)-N-(3-(difluoromethoxy)-5-fluorobenzyl)-1-(4-fluorophenylsulfonyl) pyrrolidine-2-carboxamide

was prepared starting from (S)-1-(4-fluorophenylsulfonyl)pyrrolidine-2-carboxylic acid (0.285 mmol, 0.078 g) and (3-(difluoromethoxy)-5-fluorophenyl)methanamine (0.314 mmol, 0.06 g). The product was purified by preparative HPLC. 27 mg of the title compound was obtained.
¹H NMR (400 MHz, CDCl₃) δ ppm 1.62 - 1.88 (m, 3 H), 2.18 - 2.28 (m, 1 H), 3.13 - 3.23 (m, 1 H), 3.57 - 3.66 (m, 1 H), 4.12 (dd, 1 H), 4.39 - 4.64 (m, 2 H), 6.37 - 6.80 (m, 2 H), 6.86 - 6.94 (m, 2 H), 7.27 (m, 2 H), 7.34 - 7.45 (m, 1 H), 7.83 - 7.94 (m, 2 H).

### EXAMPLE 14: (2S,4R)-1-(3,4-Difluorophenylsulfonyl)-4-fluoro-N-((2-(3,3,3-trifluoropropoxy)pyridin-4-yl)methyl)pyrrolidine-2-carboxamide

was prepared starting from (2S,4R)-1-(3,4-difluorophenylsulfonyl)-4-fluoropyrrolidine-2-carboxylic acid (0.323 mmol, 0.100 g) and (2-(3,3,3-trifluoropropoxy)pyridin-4-yl)methanamine hydrochloride (0.323 mmol, 0.083 g). The product was purified using preparative HPLC. 0.100 g of the title compound was obtained.
¹H NMR (400 MHz, CDCl₃ δ ppm 2.16 - 2.36 (m, 1 H) 2.46 - 2.67 (m, 3 H) 3.53 - 3.70 (m, 1 H) 3.81 - 3.94 (m, 1 H) 4.29 (t, 1 H) 4.35 - 4.56 (m, 4 H) 4.97 - 5.14 (m, 1 H) 6.69 (dd, 1 H) 6.85 (dd, 1 H) 7.29 - 7.37 (m, 1 H) 7.40 (t, 1 H) 7.61 - 7.66 (m, 1 H) 7.67 - 7.73 (m, 1 H) 8.07 (dd, 1 H).

### EXAMPLE 15: (S)-N-(3-ethoxy-5-fluorobenzyl)-1-(4-fluorophenylsulfonyl)pyrrolidine -2-carboxamide

was prepared starting from (S)-1-(4-fluorophenylsulfonyl)pyrrolidine-2-carboxylic acid (0.366 mmol, 100 mg) and (3-ethoxy-5-fluorophenyl)methanamine (0.403 mmol, 68.1 mg). The product was purified with preparative HPLC. 114 mg of the title compound was obtained.
¹H NMR (400 MHz, CDCl₃) δ ppm 1.42 (t, 3 H), 1.62 - 1.74 (m, 2 H), 1.75 - 1.89 (m, 1 H), 2.19 - 2.29 (m, 1 H), 3.14 - 3.24 (m, 1 H), 3.55 - 3.64 (m, 1 H), 4.04 (q, 2 H), 4.15 (dd, 1 H), 4.39 - 4.56 (m, 2 H), 6.50 - 6.64 (m, 2 H), 6.65 - 6.70 (m, 1 H), 7.23 - 7.35 (m, 3 H), 7.86 - 7.94 (m, 2 H).

### EXAMPLE 16: (S)-N-(3-chloro-5-methoxybenzyl)-1-(4-fluorophenylsulfonyl) pyrrolidine-2-carboxamide

was prepared starting from (S)-1-(4-fluorophenylsulfonyl)pyrrolidine-2-carboxylic acid (0.293 mmol, 80.0 mg) and (3-chloro-5-methoxyphenyl)methanamine (0.322 mmol, 55.3 mg). The product was purified using preparative HPLC. 47 mg of the title compound was obtained.
¹H NMR (400 MHz, CDCl₃) δ ppm 1.60 - 1.72 (m, 2 H) 1.73 - 1.86 (m, 1 H) 2.18 - 2.27 (m, 1 H) 3.11 - 3.21 (m, 1 H) 3.54 - 3.62 (m, 1 H) 3.79 (s, 3 H) 4.12 (dd, 1 H) 4.34 - 4.56 (m, 2 H) 6.76 - 6.81 (m, 2 H) 6.87 (t, 1 H) 7.21 - 7.29 (m, 3 H) 7.84 - 7.90 (m, 2 H).

### EXAMPLE 17: (S)-1-(Phenylsulfonyl)-N-(4-(trifluoromethyl)benzyl)pyrrolidine-2-carboxamide

was prepared starting from (S)-1-(phenylsulfonyl)pyrrolidine-2-carboxylic acid (20 mmol, 5.11 g) and 4-(trifluoromethyl)benzylamin (20.00 mmol, 3.50 g). The product was purified by recrystallization from heptane/ethyl acetate. 5.5 g of the title compound was obtained.
¹H NMR (400 MHz, CDCl₃) δ ppm 1.54 - 1.68 (m, 2 H), 1.69 - 1.83 (m, 1 H), 2.17 - 2.28 (m, 1 H), 3.15 - 3.26 (m, 1 H), 3.54 - 3.63 (m, 1 H), 4.16 (dd, 1 H), 4.48 - 4.67 (m, 2 H), 7.34 - 7.42 (m, 1 H), 7.42 - 7.48 (m, 2 H), 7.54 - 7.64 (m, 4 H), 7.64 - 7.71 (m, 1 H), 7.81 - 7.91 (m, 2 H).

### EXAMPLE 18: (2S,4R)-N-(3-(difluoromethoxy)-5-fluorobenzyl)-4-fluoro-1-(4-fluorophenylsulfonyl)pyrrolidine-2-carboxamide

was prepared starting from (2S,4R)-4-fluoro-1-(4-fluorophenylsulfonyl)pyrrolidine-2-carboxylic acid (0.275 mmol, 80.0 mg) and (3-(difluoromethoxy)-5-fluorophenyl)methanamine (0.275 mmol, 52.5 mg). The product was purified using preparative HPLC. 14 mg of the title compound was obtained.
¹H NMR (400 MHz, CDCl₃) δ ppm 2.15 - 2.35 (m, 1 H) 2.44 - 2.59 (m, 1 H) 3.53 - 3.70 (m, 1 H) 3.83 - 3.96 (m, 1 H) 4.25 (t, 1 H) 4.37 - 4.63 (m, 2 H) 4.95 - 5.12 (m, 1 H) 6.57 (br. t., 1 H) 6.73 - 6.80 (m, 1 H) 6.88 - 6.94 (m, 2 H) 7.19 - 7.26 (m, 2 H) 7.31 (t, 1 H) 7.83 - 7.91 (m, 2 H).

### EXAMPLE 19: (S)-1-(4-Fluorophenylsulfonyl)-N-((2-(isobutylthio)pyridin-4-yl)methyl)pyrrolidine-2-carboxamide

was prepared starting from (S)-1-(4-fluorophenylsulfonyl)pyrrolidine-2-carboxylic acid (2 mmol, 0.547 g) and (2-(isobutylthio)pyridin-4-yl)methanamine hydrochloride (2.000 mmol, 0.466 g). The product was purified by flash chromatography. 0.723 g of the title compound was obtained.
¹H NMR (400 MHz, CDCl₃) δ ppm 1.05 (d, 6 H), 1.62 - 1.73 (m, 2 H), 1.73 - 1.87 (m, 1 H), 1.89 - 2.01 (m, 1 H), 2.20 - 2.30 (m, 1 H), 3.09 (d, 2 H), 3.13 - 3.22 (m, 1 H), 3.57 - 3.64 (m, 1 H), 4.09 - 4.17 (m, 1 H), 4.36 - 4.54 (m, 2 H), 6.91 (d, 1 H), 7.12 (s, 1 H), 7.23 - 7.34 (m, 3 H), 7.86 - 7.93 (m, 2 H), 8.37 (d, 1 H).

### EXAMPLE 20: (S)-N-(4-chlorobenzyl)-1-(4-fluorophenylsulfonyl)pyrrolidine-2-carboxamide

was prepared starting from (S)-1-(4-fluorophenylsulfonyl)pyrrolidine-2-carboxylic acid (1.3 mmol, 0.355 g) and 4-chlorobenzylamine (1.300 mmol, 0.184 g). The product was purified by flash chromatography. 0.418 g of the title compound was obtained.
¹H NMR (400 MHz, CDCl₃) δ ppm 1.59 - 1.72 (m, 2 H), 1.72 - 1.84 (m, 1 H), 2.20 - 2.29 (m, 1 H), 3.12 - 3.21 (m, 1 H), 3.53 - 3.61 (m, 1 H), 4.11 (dd, 1 H), 4.41 - 4.56 (m, 2 H), 7.17 - 7.36 (m, 7 H), 7.84 - 7.91 (m, 2 H).

### EXAMPLE 21: (2S,4R)-N-(4-chloro-3-(isopropylamino)benzyl)-4-fluoro-1-(phenylsulfonyl)pyrrolidine-2-carboxamide

was prepared starting from (2S,4R)-4-fluoro-1-(phenylsulfonyl)pyrrolidine-2-carboxylic acid (1.05 mmol, 0.287 g) and 5-(aminomethyl)-2-chloro-N-isopropylaniline (1.26 mmol, 0.250 g). The product was purified using preparative HPLC. 0.128 g of the title compound was obtained.
¹H NMR (400 MHz, CDCl₃) δ ppm 1.25 (d, 6 H) 2.15 - 2.35 (m, 1 H) 2.37 - 2.55 (m, 1 H) 3.49 - 3.66 (m, 1 H) 3.66 - 3.78 (m, 1 H) 3.81 - 3.95 (m, 1 H) 4.15 (d, 1 H) 4.28 (t, 1 H) 4.34 - 4.50 (m, 2 H) 4.88 - 5.11 (m, 1 H) 6.51 - 6.56 (m, 1 H) 6.66 (bs, 1 H) 7.14 - 7.22 (m, 2 H) 7.49 - 7.58 (m, 2 H) 7.60 - 7.68 (m, 1 H) 7.81 - 7.87 (m, 2 H).

### EXAMPLE 22: (S)-N-(3-cyclopropylbenzyl)-1-(phenylsulfonyl)pyrrolidine-2-carboxamide

was prepared starting from (S)-1-(phenylsulfonyl)pyrrolidine-2-carboxylic acid (0.220 mmol, 0.056 g) and (3-cyclopropylphenyl)methanamine hydrochloride (0.253 mmol, 0.046 g). The product was purified by flash chromatography. 0.059 g of the title compound was obtained. ¹H NMR (400 MHz, CDCl₃) δ ppm 0.65 - 0.79 (m, 2 H) 0.91 - 0.99 (m, 2 H) 1.52 - 1.83 (m, 3 H) 1.84 - 1.94 (m, 1 H) 2.17 - 2.28 (m, 1 H) 3.14 - 3.25 (m, 1 H) 3.50 - 3.59 (m, 1 H) 4.16(dd, 1 H) 4.47 (d, 2 H) 6.96 - 7.11 (m, 3 H) 7.16 - 7.26 (m, 2 H) 7.52 - 7.60 (m, 2 H) 7.61 - 7.69 (m, 1 H) 7.82 - 7.88 (m, 2 H).

### EXAMPLE 23: (S)-N-(3-cyclopropylbenzyl)-1-(4-fluorophenylsulfonyl)pyrrolidine-2-carboxamide

was prepared starting from (S)-1-(4-fluorophenylsulfonyl)pyrrolidine-2-carboxylic acid (0.220 mmol, 0.060 g) and (3-cyclopropylphenyl)methanamine hydrochloride (0.253 mmol, 0.046 g). The product was purified by flash chromatography. 0.064 g of the title compound was obtained.
¹H NMR (400 MHz, CDCl₃) δ ppm 0.68 - 0.74 (m, 2 H) 0.92 - 0.98 (m, 2 H) 1.58 - 1.85 (m, 3 H) 1.85 - 1.94 (m, 1 H) 2.19 - 2.29 (m, 1 H) 3.11 - 3.21 (m, 1 H) 3.50 - 3.58 (m, 1 H) 4.10 - 4.16 (m, 1 H) 4.46 (d, 2 H) 6.97 - 7.03 (m, 2 H) 7.05 - 7.10 (m, 1 H) 7.13 (bs, 1 H) 7.20 - 7.26 (m, 3 H) 7.83 - 7.89 (m, 2 H).

### EXAMPLE 24: (S)-1-(Phenylsulfonyl)-N-(3-(trifluoromethyl)benzyl)pyrrolidine-2-carboxamide

was prepared starting from (S)-1-(phenylsulfonyl)pyrrolidine-2-carboxylic acid (0.4 mmol, 102 mg) and 3-(trifluoromethyl)benzylamine (0.400 mmol, 70.1 mg). The product was purified by flash chromatography. 80 mg of the title compound was obtained.
¹H NMR (400 MHz, CDCl₃) δ ppm 1.59 - 1.69 (m, 2 H), 1.70 - 1.83 (m, 1 H), 2.20 - 2.29 (m, 1 H), 3.17 - 3.26 (m, 1 H), 3.55 - 3.63 (m, 1 H), 4.13 - 4.20 (m, 1 H), 4.52 - 4.65 (m, 2 H), 7.34 - 7.42 (m, 1 H), 7.45 - 7.62 (m, 6 H), 7.64 - 7.71 (m, 1 H), 7.83 - 7.90 (m, 2 H).

### EXAMPLE 25: (S)-1-(Phenylsulfonyl)-N-(3-pentafluorothio)benzyl)pyrrolidine-2-carboxamide

was prepared starting from (S)-1-(phenylsulfonyl)pyrrolidine-2-carboxylic acid (0.274 mmol, 70.0 mg) and (3-(pentafluorothio)phenyl)methanamine (0.302 mmol, 70.3 mg). The product was purified using preparative HPLC. 41 mg of the title compound was obtained.
¹H NMR (400 MHz, CDCl₃) δ ppm 1.54 - 1.83 (m, 3 H) 2.15 - 2.27 (m, 1 H) 3.13 - 3.26 (m, 1 H) 3.54 - 3.64 (m, 1 H) 4.15 (dd, 1 H) 4.57 (d, 2 H) 7.39 - 7.53 (m, 3 H) 7.53 - 7.62 (m, 2 H) 7.62 - 7.72 (m, 3 H) 7.85 (d, 2 H).

### EXAMPLE 26: (2S,4R)-4-Fluoro-1-(4-fluorophenylsulfonyl)-N-((2-(3,3,3-trifluoropropoxy)pyridin-4-yl)methyl)pyrrolidine-2-carboxamide

(2S,4R)-4-fluoro-1-(4-fluorophenylsulfonyl)-N-((2-(3,3,3-trifluoropropoxy)pyridin-4-yl)methyl)pyrrolidine-2-carboxamide was prepared starting from (2S,4R)-4-fluoro-1-((4-fluorophenyl)sulfonyl)pyrrolidine-2-carboxylic acid (0.343 mmol, 0.100 g) and (2-(3,3,3-trifluoropropoxy)pyridin-4-yl)methanamine hydrochloride (0.343 mmol, 0.088 g). The product was purified using preparative HPLC. 0.068 g of the title compound was obtained. ¹H NMR (400 MHz, CDCl₃) δ ppm 2.17 - 2.34 (m, 1 H) 2.43 - 2.54 (m, 1 H) 2.54 - 2.67 (m, 2 H) 3.54-3.71 (m, 1 H) 3.82 - 3.94 (m, 1 H) 4.27 (t, 1 H) 4.36 - 4.56 (m, 4 H) 4.94 - 5.12 (m, 1 H) 6.70 (dd, 1 H) 6.86 (dd, 1 H) 7.17 - 7.25 (m, 2 H) 7.44 (t, 1 H) 7.83 - 7.89 (m, 2 H) 8.08 (dd, 1 H).

### EXAMPLE 27: (S)-1-(4-Fluorophenylsulfonyl)-N-((4-(trifluoromethyl)pyridin-2-yl)methyl)pyrrolidine-2-carboxamide

was prepared starting from (S)-1-(4-fluorophenylsulfonyl)pyrrolidine-2-carboxylic acid (0.293 mmol, 80.0 mg) and (4-(trifluoromethyl)pyridin-2-yl)methanamine hydrochloride (0.322 mmol, 68.5 mg). The product was purified using preparative HPLC. 66 mg of the title compound was obtained.
¹H NMR (400 MHz, CDCl₃) δ ppm 1.62 - 1.76 (m, 2 H) 1.77 - 1.92 (m, 1 H) 2.16 - 2.27 (m, 1 H) 3.15 - 3.26 (m, 1 H) 3.58 - 3.67 (m, 1 H) 4.18 (dd, 1 H) 4.63 - 4.80 (m, 2 H) 7.21 - 7.28 (m, 2 H) 7.43 (d, 1 H) 7.58 (s, 1 H) 7.82 (t, 1 H) 7.87 - 7.94 (m, 2 H) 8.75 (d, 1 H).

### EXAMPLE 28: (S)-1-(4-Fluorophenylsulfonyl)-N-((5-(isopentyloxy)pyridin-3-yl)methyl) pyrrolidine-2-carboxamide

was prepared starting from (S)-1-(4-fluorophenylsulfonyl)pyrrolidine-2-carboxylic acid (0.273 mmol, 75.0 mg) and (5-(isopentyloxy)pyridin-3-yl)methanamine hydrochloride (0.273 mmol, 63.0 mg). The product was purified using preparative HPLC. 55.5 mg of the title compound was obtained.
¹H NMR (400 MHz, CDCl₃) δ ppm 0.95 (d, 6 H) 1.60 - 1.89 (m, 6 H) 2.16 - 2.25 (m, 1 H) 3.11 - 3.20 (m, 1 H) 3.53 - 3.62 (m, 1 H) 4.02 - 4.07 (m, 2 H) 4.10 (dd, 1 H) 4.40 - 4.64 (m, 2 H) 7.21 - 7.29 (m, 3 H) 7.32 (t, 1 H) 7.83 - 7.90 (m, 2 H) 8.13 (d, 1 H) 8.21 (d, 1 H).

### EXAMPLE 29: (2S,4R)-4-Fluoro-1-(4-fluorophenylsulfonyl)-N-((5-(isopentyloxy) pyridine-3-yl)methyl)pyrrolidine-2-carboxamide

was prepared starting from (2S,4R)-4-fluoro-1-(4-fluorophenylsulfonyl)pyrrolidine-2-carboxylic acid (0.273 mmol, 80.0 mg) and (5-(isopentyloxy)pyridin-3-yl)methanamine hydrochloride (0.273 mmol, 63.0 mg). The product was purified using preparative HPLC. 36.9 mg of the title compound was obtained.
¹H NMR (400 MHz, CDCl₃) δ ppm 0.95 (d, 6 H) 1.68 (q, 2 H) 1.76 - 1.88 (m, 1 H) 2.14-2.33 (m, 1 H) 2.45 - 2.59 (m, 1 H) 3.53 - 3.70 (m, 1 H) 3.81 - 3.93 (m, 1 H) 4.05 (t, 2 H) 4.25 (t, 1 H) 4.40 - 4.64 (m, 2 H) 4.95 - 5.12 (m, 1 H) 7.18 - 7.25 (m, 2 H) 7.29 (dd, 1 H) 7.35 (t, 1 H) 7.83 - 7.89 (m, 2 H) 8.14 (d, 1 H) 8.20 (d, 1 H).

### EXAMPLE 30: (S)-1-(4-Fluorophenylsulfonyl)-N-((4-isobutoxypyridin-2-yl)methyl) pyrrolidine-2-carboxamide

was prepared starting from (S)-1-(4-fluorophenylsulfonyl)pyrrolidine-2-carboxylic acid (0.366 mmol, 100.0 mg) and (4-isobutoxypyridin-2-yl)methanamine hydrochloride (0.366 mmol, 79.0 mg). The product was purified using preparative HPLC. 49.2 mg of the title compound was obtained.
¹H NMR (400 MHz, CDCl₃) δ ppm 1.01 (d, 6 H) 1.61 - 1.92 (m, 3 H) 2.04 - 2.18 (m, 2 H) 3.15 - 3.24 (m, 1 H) 3.58 - 3.66 (m, 1 H) 3.84 (d, 2 H) 4.15 (dd, 1 H) 4.48 - 4.75 (m, 2 H) 6.80 (dd, 1 H) 7.01 (d, 1 H) 7.19 - 7.26 (m, 2 H) 7.85 - 7.92 (m, 2 H) 7.97 (t, 1 H) 8.36 (d, 1 H).

### EXAMPLE 31: (S)-1-(4-Fluorophenylsulfonyl)-N-((4-(isobutylthio)pyridin-2-yl)methyl) pyrrolidine-2-carboxamide

was prepared starting from (S)-1-(4-fluorophenylsulfonyl)pyrrolidine-2-carboxylic acid (0.329 mmol, 90.0 mg) and (4-isobutylthio)pyridin-2-yl)methanamine hydrochloride (0.329 mmol, 81.0 mg). The product was purified by flash chromatography. 29.5 mg of the title compound was obtained.
¹H NMR (400 MHz, CDCl₃) δ ppm 1.06 (d, 6 H) 1.60 - 1.77 (m, 2 H) 1.78 - 2.01 (m, 2 H) 2.16-2.26(m, 1 H)2.88(d,2H)3.17-3.27(m, 1 H) 3.56 - 3.69 (m, 1 H) 4.18 (dd, 1 H) 4.46 - 4.66 (m, 2 H) 7.01 (dd, 1 H) 7.13 (d, 1 H) 7.20 - 7.27 (m, 2 H) 7.68 (br. s., 1 H) 7.87 - 7.94 (m, 2 H) 8.31 (d, 1 H).

### EXAMPLE 32: (S)-1-(3,4-Difluorophenylsulfonyl)-N-((4-(2,2,2-trifluoroethoxy) pyridin-2-yl)methyl)pyrrolidine-2-carboxamide

was prepared starting from (S)-1-((3,4-difluorophenyl)sulfonyl)pyrrolidine-2-carboxylic acid (0.292 mmol, 85.0 mg) and (4-(2,2,2-trifluoroethoxy)pyridin-2-yl)methanamine hydrochloride (0.292 mmol, 71.0 mg). The product was purified by flash chromatography. 48.7 mg of the title compound was obtained.
¹H NMR (400 MHz, CDCl₃) δ ppm 1.68 - 1.97 (m, 3 H) 2.14 - 2.25 (m, 1 H) 3.15 - 3.25 (m, 1 H) 3.58 - 3.66 (m, 1 H) 4.18 (dd, 1 H) 4.39 - 4.51 (m, 3 H) 4.78 (dd, 1 H) 6.79 (dd, 1 H) 6.98 (d, 1 H) 7.34 - 7.42 (m, 1 H) 7.62 (t, 1 H) 7.65 - 7.70 (m, 1 H) 7.71 - 7.78 (m, 1 H) 8.40 (d, 1 H).

### EXAMPLE 33: (S)-1-(3,5-Difluorophenylsulfonyl)-N-((4-(2,2,2-trifluoroethoxy) pyridine-2-yl)methyl)pyrrolidine-2-carboxamide

was prepared starting from (S)-1-(3,5-difluorophenylsulfonyl)pyrrolidine-2-carboxylic acid (0.292 mmol, 85.0 mg) and (4-(2,2,2-trifluoroethoxy)pyridin-2-yl)methanamine hydrochloride (0.292 mmol, 71.0 mg). The product was purified by flash chromatography. 49.1 mg of the title compound was obtained.
¹H NMR (400 MHz, CDCl₃) δ ppm 1.70 - 1.99 (m, 3 H) 2.17 - 2.26 (m, 1 H) 3.18 - 3.28 (m, 1 H) 3.59 - 3.68 (m, 1 H) 4.21 (dd, 1 H) 4.39 - 4.51 (m, 3 H) 4.78 (dd, 1 H) 6.79 (dd, 1 H) 6.97 (d, 1 H) 7.08 - 7.15 (m, 1 H) 7.39 - 7.47 (m, 2 H) 7.60 (t, 1 H) 8.41 (d, 1 H).

### EXAMPLE 34: (2S,4R)-N-(4-cyano-3-propylbenzyl)-4-fluoro-1-(phenylsulfonyl) pyrrolidine-2-carboxamide

PS-Carbodiimide (0.350 mmol, 0.263 g) was weighed in a vial. (2S,4R)-4-Fluoro-1-(phenylsulfonyl)pyrrolidine-2-carboxylic acid (0.200 mmol, 0.055 g) in dichloromethane (3 ml) was added. The mixture was shaked for 10 minutes. 4-(aminomethyl)-2-propylbenzonitrile (0.150 mmol, 0.026 g) was added. The mixture was shaked over night. The solids were filtered off. The filtrate was washed with 1M NaOH and 1M HCl. The organic phase was evaporated to dryness. 356 mg of the title compound was obtained.
¹H NMR (400 MHz, CDCl₃) δ ppm 0.99 (t, 3 H), 1.67 - 1.78 (m, 2 H), 2.16 - 2.35 (m, 1 H), 2.41 - 2.55 (m, 1 H), 2.78 - 2.86 (m, 2 H), 3.53 - 3.70 (m, 1 H), 3.84 - 3.97 (m, 1 H), 4.31 (t, 1 H), 4.45 (dd, 1 H), 4.63 (dd, 1 H), 4.93 - 5.11 (m, 1 H), 7.23 - 7.29 (m, 1 H), 7.31 - 7.34 (m, 1 H), 7.34 - 7.41 (m, 1 H), 7.52 - 7.61 (m, 3 H), 7.63 - 7.70 (m, 1 H), 7.83 - 7.88 (m, 2 H).

### EXAMPLE 35: (S)-N-(4-cyano-3-isopropylbenzyl)-1-(phenylsulfonyl)pyrrolidine-2-carboxamide

was prepared using a procedure similar to the one described for ((2S,4R)-N-(4-cyano-3-propylbenzyl)-4-fluoro-1-(phenylsulfonyl)pyrrolidine-2-carboxamide, starting from (S)-1-(phenylsulfonyl)pyrrolidine-2-carboxylic acid (0.300 mmol, 0.077 g) and 4-(aminomethyl)-2-isopropylbenzonitrile hydrochloride (0.200 mmol, 0.042 g). Flash chromatography gave 44 mg of the title compound.
¹H NMR (400 MHz, CDCl₃) δ ppm 1.33 (d, 6 H) 1.56 - 1.83 (m, 3 H) 2.17 - 2.26 (m, 1 H) 3.14 - 3.23 (m, 1 H) 3.33 - 3.42 (m, 1 H) 3.54 - 3.64 (m, 1 H) 4.09 - 4.20 (m, 1 H) 4.41-4.72 (m, 2 H) 7.20 - 7.25 (m, 1 H) 7.34 - 7.42 (m, 2 H) 7.55 - 7.62 (m, 3 H) 7.64 - 7.71 (m, 1 H) 7.83 - 7.89 (m, 2 H).

### EXAMPLE 36: (2S,4R)-4-Fluoro-1-(4-fluorophenylsulfonyl)-N-((2-(isobutylthio) pyridin-4-yl)methyl)pyrrolidine-2-carboxamide

was prepared using a procedure similar to the one described for ((2S,4R)-N-(4-cyano-3-propylbenzyl)-4-fluoro-1-(phenylsulfonyl)pyrrolidine-2-carboxamide, starting from (2S,4R)-4-fluoro-1-(4-fluorophenylsulfonyl)pyrrolidine-2-carboxylic acid (0.250 mmol, 0.073 g) and (2-(isobutylthio)pyridin-4-yl)methanamine. 0.016 g of the title compound was obtained.
¹H NMR (400 MHz, CDCl₃) δ ppm 1.04 (d, 6 H) 1.87 - 2.01 (m, 1 H) 2.17 - 2.36 (m, 1 H) 2.44 - 2.61 (m, 1 H) 3.09 (d, 2 H) 3.53 - 3.70 (m, 1 H) 3.84 - 3.96 (m, 1 H) 4.26 (t, 1 H) 4.33 - 4.55 (m, 2 H) 4.95 - 5.13 (m, 1 H) 6.91 - 6.94 (m, 1 H) 7.14 (dd, 1 H) 7.19 - 7.26 (m, 2 H) 7.31 (t, 1 H) 7.85 - 7.90 (m, 2 H) 8.36 (dd, 1 H).

### EXAMPLE 37: (S)-N-(4-bromobenzyl)-1-(4-fluorophenylsulfonyl)pyrrolidine-2-carboxamide

PS-Carbodiimide (1.220 mmol, 0.917 g) was weighed in a vial. (S)-1-(4-fluorophenyl sulfonyl)pyrrolidine-2-carboxylic acid (0.915 mmol, 0.25 g) in dichloromethane (10 ml) was added. The resulting mixture was stirred at room temperature for 10 minutes. p-Bromobenzylamine (0.610 mmol, 0.113 g) was added. The mixture was stirred overnight. The crude product was filtered, the solids were washed with dichloromethane and the filtrate was evaporated. The product was purified using silica plug filtration and recrystallization from diethyl ether, respectively. 100.14 mg of the title compound was obtained.
¹H NMR (400 MHz, DMSO-*d*₆) δ ppm 1.47 - 1.58 (m, 1 H), 1.70 - 1.85 (m, 3 H), 3.13 - 3.23 (m, 1 H), 3.42 - 3.50 (m, 1 H), 4.02 - 4.10 (m, 1 H), 4.20 - 4.34 (m, 2 H), 7.24 (d, 2 H), 7.43 - 7.54 (m, 4 H), 7.90 - 7.98 (m, 2 H), 8.55 - 8.63 (m, 1 H).

### EXAMPLE 38: (S)-N-(4-ethylbenzyl)-1-(4-fluorophenylsulfonyl)pyrrolidine-2-carboxamide

### Step 1: (S)-1-(4-Fluorophenylsulfonyl)pyrrolidine-2-carbonyl chloride.

(S)-1-(4-Fluorophenylsulfonyl)pyrrolidine-2-carboxylic acid (5.49 mmol, 1.5 g) was dissolved in dry toluene (20 ml). Thionyl chloride (8.23 mmol, 0.980 g) was added dropwise. The resulting mixture was stirred at room temperature for an hour. The solvent was evaporated. 1.8 g of the title compound was obtained as a yellow powder.

### Step 2: (S)-N-(4-ethylbenzyl)-1-(4-fluorophenylsulfonyl)pyrrolidine-2-carboxamide

4-Ethylbenzylamine (0.686 mmol, 0.093 g) and sodium carbonate (1.371 mmol, 0.145 g) were dissolved in water (20 ml). (S)-1-(4-fluorophenylsulfonyl)pyrrolidine-2-carbonyl chloride (0.686 mmol, 0.2 g), was added and the resulting mixture was stirred overnight at room temperature. Water was evaporated. The residue was dissolved in ethyl acetate and washed twice with 2M NaOH and once with 2M HCl and water. The product was dried and evaporated with vacuum. 160 mg of the title compound was obtained.
¹H NMR (400 MHz, DMSO-*d*₆) δ ppm 1.16 (t, 3 H), 1.48 - 1.58 (m, 1 H), 1.69 - 1.85 (m, 3 H), 2.58 (q, 2 H), 3.13 - 3.23 (m, 1 H), 3.41 - 3.50 (m, 1 H), 4.08 (dd, 1 H), 4.18 - 4.34 (m, 2 H), 7.12 - 7.22 (m, 4 H), 7.42 - 7.51 (m, 2 H), 7.89 - 7.97 (m, 2 H), 8.43 - 8.51 (m, 1 H).

### EXAMPLE 39: (S)-1-(4-Fluorophenylsulfonyl)-N-(3-(propylamino)benzyl)pyrrolidine-2-carboxamide

### Step 1: (S)-N-(3-aminobenzyl)-1-(4-fluorophenylsulfonyl)pyrrolidine-2-carboxamide

was prepared using a procedure similar to the one described for (S)-1-(4-fluorophenylsulfonyl)-N-(3-(2-(methylthio)ethoxy)benzyl)pyrrolidine-2-carboxamide in Example 1, starting from (S)-1-(4-fluorophenylsulfonyl)pyrrolidine-2-carboxylic acid (18.30 mmol, 5.000 g) and 3-aminobenzylamine (18.30 mmol, 2.235 g). The product was purified by flash chromatography. 4.48 g of the title compound was obtained.
¹H NMR (400 MHz, CDCl₃) δ ppm 1.58 - 1.70 (m, 2 H) 1.70 - 1.85 (m, 1 H) 2.18 - 2.27 (m, 1 H) 3.12 - 3.21 (m, 1 H) 3.50 - 3.58 (m, 1 H) 3.70 (bs, 2 H) 4.13 (dd, 1 H) 4.40 (d, 2 H) 6.57 - 6.62 (m, 1 H) 6.64 - 6.70 (m, 2 H) 7.07 - 7.15 (m, 2 H) 7.19 - 7.26 (m, 2 H) 7.83 - 7.90 (m, 2 H).

### Step 2: (S)-1-(4-Fluorophenylsulfonyl)-N-(3-(propylamino)benzyl)pyrrolidine-2-carboxamide.

(S)-N-(3-aminobenzyl)-1-(4-fluorophenylsulfonyl)pyrrolidine-2-carboxamide (2.65 mmol, 1.000 g) and 1,2-dichloroethane (28 ml) were stirred in an flask. Acetic acid, glacial (3.97 mmol, 0.239 g) was added and the mixture was cooled by an ice bath. Sodium triacetoxy borohydride (5.30 mmol, 1.123 g) was added. The resulting mixture was stirred at room temperature for one hour. Water was added and after stirring for 5 minutes the phases were separated. The organic phase was washed with saturated NaHCO₃ -solution and brine, dried and evaporated. The product was purified by flash chromatography. 0.420 g of the title compound was obtained.
¹H NMR (400 MHz, CDCl₃) δ ppm 0.99 (t, 3 H) 1.58 - 1.70 (m, 4 H) 1.71 - 1.85 (m, 1 H) 2.19 - 2.28 (m, 1 H) 3.06 - 3.12 (m, 2 H) 3.12 - 3.21 (m, 1 H) 3.50 - 3.57 (m,1 H) 3.69 (bs, 1 H) 4.10 - 4.16 (m, 1 H) 4.42 (d, 2 H) 6.49 - 6.54 (m, 1 H) 6.55 - 6.58 (m, 1 H) 6.58 - 6.63 (m, 1 H) 7.08 (bs, 1 H) 7.14 (t, 1 H) 7.20 - 7.26 (m, 2 H) 7.83 - 7.89 (m, 2 H).

As already mentioned hereinbefore, the compounds of formula I show interesting pharmacological properties, namely they exhibit TRPA1 activity. The said activity is demonstrated with the pharmacological test presented below.

### EXPERIMENT 1: Determination of TRPA1 activity in vitro

The illustrative examples of the present invention were screened for TRPA1 activity according to a procedure described in Wei et al., Anesthesiology 111 (2009) 147-154. The results are shown in Table 1.

**Table 1. TRPA1 antagonism in vitro.**

| Compound of example | IC50 (µM) | Compound of example | IC50 (µM) | Compound of example | IC50 (µM) |
|---|---|---|---|---|---|
| 1 | 4.0 | 14 | 26.0 | 27 | 33.8 |
| 2 | 18.6 | 15 | 5.9 | 28 | 4.1 |
| 3 | 10.4 | 16 | 5.4 | 29 | 13.6 |
| 4 | 11.3 | 17 | 13.0 | 30 | 10.5 |
| 5 | 9.8 | 18 | 24.1 | 31 | 8.5 |
| 6 | 5.1 | 19 | 9.3 | 32 | 12.5 |
| 7 | 17.3 | 20 | 12.5 | 33 | 18.5 |
| 8 | 14.5 | 21 | 15.6 | 34 | 12.6 |
| 9 | 5.3 | 22 | 13.6 | 35 | 9.3 |
| 10 | 16.9 | 23 | 7.7 | 36 | 17.4 |
| 11 | 4.4 | 24 | 17.0 | 37 | 11.6 |
| 12 | 16.3 | 25 | 6.7 | 38 | 16.8 |
| 13 | 11.5 | 26 | 37.0 | 39 | 14.1 |

The compounds of formula I exhibit TRPA1 antagonism. The present invention thus provides compounds for use as a medicament. Compounds for use in the treatment of disorder, condition or disease mediated by TRPA1 receptor activity are also provided. Furthermore, a method for the treatment of disorder, condition or disease mediated by TRPA1 receptor activity is provided. In said method an effective amount of at least one compound of formula I is administered to a mammal, e.g. human, in need of such treatment. The use of the compounds of formula I for the manufacture of a medicament for the treatment of disorder, condition or disease mediated by TRPA1 receptor activity is also provided.

In one embodiment of the invention the aforementioned disorder, condition or disease mediated by TRPA1 receptor activity is asthma, cough, allodynia, chronic obstructive pulmonary disease (COPD), tear gas irritation, pain in diabetic polyneuropathy, sleep deprivation-induced pain, sleep deprivation-induced allodynia, neurogenic inflammation, fibromyalgia, pruritus in diabetes, drug-induced pruritus, insect bite-induced pruritus, itch, neurogenic itch, neuropathic itch, psychogenic itch, mechanical hypersensitivity, migraine, neuropathic pain, nerve injury-induced neuropathic pain, postherpetic neuralgia, low back pain, parkinson pain, postherpetic pain, trigeminal neuralgia, neuropathy in diabetes, environmental chemical-induced neuropathy, neuropathy in parkinson disease, alcohol-induced neuropathy, cancer drug-induced neuropathy and pain, cancer drug-induced cold hypersensitivity, diabetic autonomic neuropathy, cardiovascular autonomic neuropathy, gastrointestinal autonomic neuropathy, polydipsia, psychogenic polydipsia, nocturia, overactive urinary bladder, erectile dysfunction, sudomotor dysfunction, primary headache, dental pain, dental cold hypersensitivity, ear pain, eye pain, spinal cord injury-induced pain, poststroke pain, pancreatitis pain, inflammatory pain, visceral pain, gastric pain, abdominal pain, burn injury-induced pain and allodynia, central pain, coeliac pain, cold pain, cold hypersensitivity, frostbite-induced pain, labor pain, musculoskeletal pain, nausea, vomiting, drug-induced nausea and vomiting, radiation-induced pain and allodynia, opioid-resistant postoperative pain, acute pain, insect bite-induced pain, urticaria, hangover headache or neurocardiogenic syncope; for example neuropathic pain, pain in diabetic polyneuropathy, postoperative pain, cancer pain, migraine, asthma, cough, pain in osteoarthritis, pain in rheumatoid arthritis or inflammatory bowel disease.

The compounds of the invention can be administered, for example, enterally, topically or parenterally by means of any pharmaceutical formulation useful for said administration and comprising at least one active compound of formula I in pharmaceutically acceptable and effective amounts together with pharmaceutically acceptable diluents, carriers and/or excipients known in the art. The manufacture of such pharmaceutical formulations is known in the art.

The therapeutic dose to be given to a subject in need of the treatment will vary depending on the compound being administered, the species, the age and the sex of the subject being treated, the particular condition being treated, as well as the route and method of administration, and is easily determined by a person skilled in the art. Accordingly, the typical dosage for oral administration is from 10 ng/kg to 100 mg/kg per day and for parenteral administration from 1 ng/kg to 10 mg/kg for an adult mammal.

The compounds of the invention are given to the subject as such or in combination with one or more other active ingredients, each in its own composition or some or all of the active ingredients combined in a single composition, and/or suitable pharmaceutical excipients. Suitable pharmaceutical excipients include conventionally used excipients and formulation aids, such as fillers, binders, disintegrating agents, lubricants, solvents, gel forming agents, emulsifiers, stabilizers, colorants and/or preservatives.

The compounds of the invention are formulated into dosage forms using commonly known pharmaceutical manufacturing methods. The dosage forms can be, for example, tablets, capsules, granules, suppositories, emulsions, suspensions or solutions. Depending on the route of administration and the galenic form, the amount of the active ingredient in a formulation can typically vary between 0.01 % and 100 % by weight.

A person skilled in the art will appreciate that the embodiments described in this application can be modified without departing from the inventive concept. A person skilled in the art also understands that the invention is not limited to the particular embodiments disclosed but is intended to also cover modifications of the embodiments that are within the scope of the invention.

## Claims

1. A compound of Formula I, 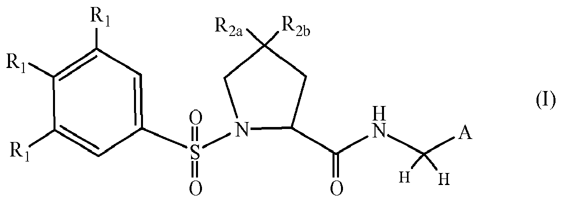 wherein A is 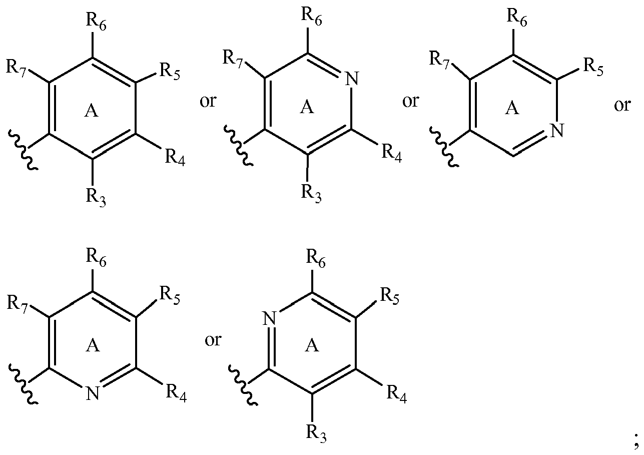 R₁ is, independently at each occurrence, H or F provided that at least one of R₁ is H; R₂ₐ and R_{2b} are independently H or F;
R₃ is H or F;
R₄ is H, halogen, (C₁-C₆)alkyl, (C₁-C₆)alkoxy, fluoro(C₁-C₆)alkyl, fluoro(C₁-C₆)alkoxy, (C₁-C₆)alkyl-(C=O)-, (C₁-C₆)alkylamino, fluoro(C₁-C₆)alkylamino or CN;
R₅ is H, halogen, (C₁-C₄)alkyl, (C₁-C₃)alkoxy, fluoro(C₁-C₃)alkyl, fluoro(C₁-C₃)alkoxy, (C₁-C₃)alkylamino, CN or SF₅;
R₆ is H, halogen, (C₁-C₆)alkyl, cyclo(C₃-C₆)alkyl, (C₁-C₆)alkoxy, (C₁-C₆)alkoxy(C₁-C₆)alkoxy, fluoro(C₁-C₆)alkyl, fluoro(C₁-C₆)alkoxy, (C₁-C₆)alkyl-S-, (C₁-C₆)alkyl-S-(C₁-C₆)alkoxy, (C₁-C₆)alkyl-(C=O)-, fluoro(C₁-C₆)alkyl(C=O)-, (C₁-C₆)alkylamino, fuoro(C₁-C₆)alkylamino, fluoro(C₁-C₆)alkyl-S-, CN or SF₅; R₇ is H or F;
or a pharmaceutically acceptable salt or ester thereof;
with the provisos that the compound is not 1-((4-fluorophenyl)sulfonyl)-N-((6-methoxypyridin-3-yl)methyl)pyrrolidine-2-carboxamide, 1-((4-fluorophenyl)sulfonyl)-N-(pyridin-3-ylmethyl)pyrrolidine-2-carboxamide, 1-(phenylsulfonyl)-N-(pyridin-3-ylmethyl)pyrrolidine-2-carboxamide, N-benzyl-1-(phenylsulfonyl)pyrrolidine-2-carboxamide, N-(4-methoxybenzyl)-1-(phenylsulfonyl)pyrrolidine-2-carboxamide, N-(4-(tert-butyl)benzyl)-1-(phenylsulfonyl)pyrrolidine-2-carboxamide, N-(4-isopropylbenzyl)-1-(phenylsulfonyl)pyrrolidine-2-carboxamide, N-(4-(difluoromethoxy)-3-methoxybenzyl)-1-(phenylsulfonyl)pyrrolidine-2-carboxamide, 1-(phenylsulfonyl)-N-(3,4,5-trimethoxybenzyl)pyrrolidine-2-carboxamide, N-benzyl-1-((4-fluorophenyl)sulfonyl)pyrrolidine-2-carboxamide, 1-((4-fluorophenyl)sulfonyl)-N-(4-methylbenzyl)pyrrolidine-2-carboxamide, N-(4-(tert-butyl)benzyl)-1-((4-fluorophenyl)sulfonyl)pyrrolidine-2-carboxamide, 1-((4-fluorophenyl)sulfonyl)-N-(4-methoxybenzyl)pyrrolidine-2-carboxamide, 1-((4-fluorophenyl)sulfonyl)-N-(3-methoxybenzyl)pyrrolidine-2-carboxamide, N-(4-fluorobenzyl)-1-((4-fluorophenyl)sulfonyl)pyrrolidine-2-carboxamide, N-(2-fluorobenzyl)-1-((4-fluorophenyl)sulfonyl)pyrrolidine-2-carboxamide, N-(3-bromobenzyl)-1-((4-fluorophenyl)sulfonyl)pyrrolidine-2-carboxamide, 1-((4-fluorophenyl)sulfonyl)-N-(4-(trifluoromethyl)benzyl)pyrrolidine-2-carboxamide, 1-((4-fluorophenyl)sulfonyl)-N-(3-(trifluoromethyl)benzyl)pyrrolidine-2-carboxamide, N-(4-(difluoromethoxy)benzyl)-1-((4-fluorophenyl)sulfonyl)pyrrolidine-2-carboxamide or N-(4-fluoro-3-methylbenzyl)-1-((4-fluorophenyl)sulfonyl)pyrrolidine-2-carboxamide.

2. A compound according to claim 1, wherein A is 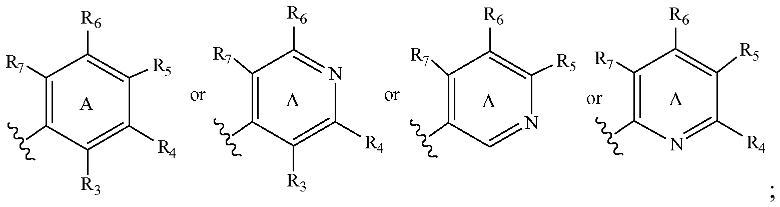 R₁ is, independently at each occurrence, H or F provided that at least one of R₁ is H; R₂ₐ and R_{2b} are independently H or F;
R₃ is H or F;
R₄ is H or halogen;
R₅ is H, halogen, (C₁-C₄)alkyl, fluoro(C₁-C₃)alkyl or CN;
R₆ is H, halogen, (C₁-C₆)alkyl, cyclo(C₃-C₆)alkyl, (C₁-C₆)alkoxy, fluoro(C₁-C₆)alkyl, fluoro(C₁-C₆)alkoxy, (C₁-C₆)alkyl-S-, (C₁-C₆)alkyl-S-(C₁-C₆)alkoxy, (C₁-C₆)alkyl-(C=O)-, (C₁-C₆)alkylamino or SF₅;
R₇ is H.

3. A compound according to any one of claims 1 or 2, wherein
R₁ is, independently at each occurrence, H or F provided that at least one of R₁ is F; R₂ₐ and R_{2b} are independently H or F;
R₃ is H or F;
R₄ is H or halogen;
R₅ is H, halogen or fluoro(C₁-C₃)alkyl;
R₆ is H, halogen, fluoro(C₁-C₆)alkyl or fluoro(C₁-C₆)alkoxy or (C₁-C₆)alkylamino; R₇ is H.

4. A compound according to any one of claims 1 to 3, wherein R₂ₐ is F and R_{2b} is H.

5. A compound according to any one of claims 1 to 3, wherein A is 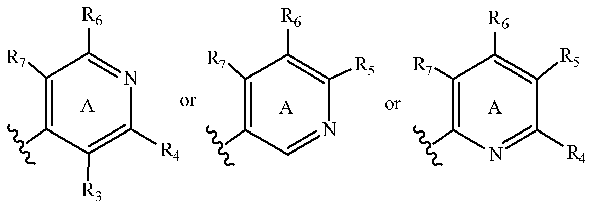 R₁ is, independently at each occurrence, H or F provided that at least one of R₁ is F; R₂ₐ and R_{2b} are independently H or F;
R₃ is H;
R₄ is H;
R₅ is H;
R₆ is fluoro(C₁-C₆)alkyl or fluoro(C₁-C₆)alkoxy;
R₇ is H.

6. A compound according to any one of claims 1 to 3, wherein A is 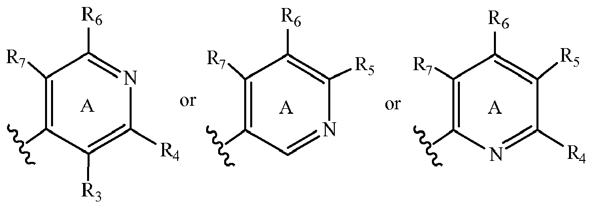 wherein
R₁ is, independently at each occurrence, H or F provided that at least one of R₁ is F; R₂ₐ is F and R_{2b} is H
R₃ is H;
R₄ is H;
R₅ is H;
R₆ is fluoro(C₁-C₆)alkyl or fluoro(C₁-C₆)alkoxy;
R₇ is H.

7. A compound according to any one of claims 1 to 3, wherein A is 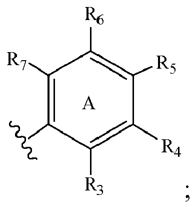 R₁ is, independently at each occurrence, H or F provided that at least one of R₁ is F; R₂ₐ and R_{2b} are independently H or F;
R₃ is H;
R₄ is H;
R₅ is fluoro(C₁-C₃)alkyl or fluoro(C₁-C₃)alkoxy;
R₆ is H;
R₇ is H.

8. A compound according to any one of claims 1 to 3, wherein A is 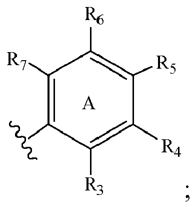 R₁ is, independently at each occurrence, H or F provided that at least one of R₁ is F; R₂ₐ is F and R_{2b} is H;
R₃ is H;
R₄ is H;
R₅ is fluoro(C₁-C₃)alkyl or fluoro(C₁-C₃)alkoxy;
R₆ is H;
R₇ is H.

9. A compound according to any one of claims 1 to 3, wherein A is 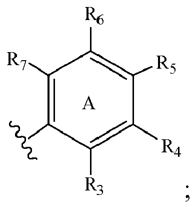 R₁ is, independently at each occurrence, H or F provided that at least one of R₁ is F; R₂ₐ and R_{2b} are independently H or F;
R₃ is H;
R₄ is H;
R₅ is H;
R₆ is (C₁-C₆)alkylamino;
R₇ is H.

10. A compound according to any one of claims 1 to 3, wherein A is 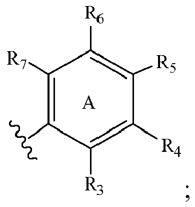 R₁ is, independently at each occurrence, H or F provided that at least one of R₁ is F; R₂ₐ is F and R_{2b} is H;
R₃ is H;
R₄ is H;
R₅ is H;
R₆ is (C₁-C₆)alkylamino;
R₇ is H.

11. A compound according to any one of claims 5 to 10, wherein R₁ is, independently at each occurrence, H or F provided that two of R₁ are F.

12. A compound according to claim 1, wherein the compound is (S)-1-(4-fluorophenylsulfonyl)-N-(3-(2-(methylthio)ethoxy)benzyl) pyrrolidine-2-carboxamide (S)-N-(3-bromobenzyl)-1-(phenylsulfonyl)pyrrolidine-2-carboxamide, (S)-N-(3-butyrylbenzyl)-1-(4-fluorophenylsulfonyl)pyrrolidine-2-carboxamide, (2S,4R)-4-fluoro-1-(4-fluorophenylsulfonyl)-N-((2-(neopentyloxy) pyridin-4-yl)methyl)pyrrolidine-2-carboxamide, (S)-N-(4-cyano-3-ethylbenzyl)-1-(4-fluorophenylsulfonyl)pyrrolidine-2-carboxamide, (S)-1-(phenylsulfonyl)-N-(3-propoxybenzyl)pyrrolidine-2-carboxamide, (S)-1-(4-fluorophenylsulfonyl)-N-((4-(2,2,2-trifluoroethoxy)pyridin-2-yl)methyl)pyrrolidine-2-carboxamide, (2S,4R)-4-fluoro-1-(4-fluorophenylsulfonyl)-N-(4-(trifluoromethyl) benzyl)pyrrolidine-2-carboxamide, (S)-N-(4-bromo-2-fluorobenzyl)-1-(4-fluorophenylsulfonyl)pyrrolidine-2-carboxamide, (S)-1-(3,5-difluorophenylsulfonyl)-N-((2-(3,3,3-trifluoropropoxy) pyridin-4-yl)methyl)pyrrolidine-2-carboxamide, (S)-N-(4-cyano-3-propylbenzyl)-1-(phenylsulfonyl)pyrrolidine-2-carboxamide, (2S,4R)-N-(4-bromo-2-fluorobenzyl)-4-fluoro-1-(4-fluorophenylsulfonyl)pyrrolidine-2-carboxamide, (S)-N-(3-(difluoromethoxy)-5-fluorobenzyl)-1-(4-fluorophenylsulfonyl)pyrrolidine-2-carboxamide, (2S,4R)-1-(3,4-difluorophenylsulfonyl)-4-fluoro-N-((2-(3,3,3-trifluoropropoxy)pyridin-4-yl)methyl)pyrrolidine-2-carboxamide, (S)-N-(3-ethoxy-5-fluorobenzyl)-1-(4-fluorophenylsulfonyl)pyrrolidine -2-carboxamide, (S)-N-(3-chloro-5-methoxybenzyl)-1-(4-fluorophenylsulfonyl)pyrrolidine-2-carboxamide, (S)- I - (phenylsulfonyl)-N-(4-(trifluoromethyl)benzyl)pyrrolidine-2-carboxamide, (2S,4R)-N-(3-(difluoromethoxy)-5-fluorobenzyl)-4-fluoro-1-(4-fluorophenylsulfonyl)pyrrolidine-2-carboxamide, (S)-1-(4-fluorophenylsulfonyl)-N-((2-(isobutylthio)pyridin-4-yl)methyl)pyrrolidine-2-carboxamide, (S)-N-(4-chlorobenzyl)-1-(4-fluorophenylsulfonyl)pyrrolidine-2-carboxamide, (2S,4R)-N-(4-chloro-3-(isopropylamino)benzyl)-4-fluoro-1-(phenylsulfonyl)pyrrolidine-2-carboxamide, (S)-N-(3-cyclopropylbenzyl)-1-(phenylsulfonyl)pyrrolidine-2-carboxamide, (S)-N-(3-cyclopropylbenzyl)-1-(4-fluorophenylsulfonyl)pyrrolidine-2-carboxamide, (S)-1-(phenylsulfonyl)-N-(3-(trifluoromethyl)benzyl)pyrrolidine-2-carboxamide, (S)-1-(phenylsulfonyl)-N-(3-pentafluorothio)benzyl)pyrrolidine-2-carboxamide, (2S,4R)-4-fluoro-1-(4-fluorophenylsulfonyl)-N-((2-(3,3,3-trifluoropropoxy)pyridin-4-yl)methyl)pyrrolidine-2-carboxamide, (S)-1-(4-fluorophenylsulfonyl)-N-((4-(trifluoromethyl)pyridin-2-yl)methyl)pyrrolidine-2-carboxamide, (S)-1-(4-fluorophenylsulfonyl)-N-((5-(isopentyloxy)pyridin-3-yl)methyl) pyrrolidine-2-carboxamide, (2S,4R)-4-fluoro-1-(4-fluorophenylsulfonyl)-N-((5-(isopentyloxy) pyridine-3-yl)methyl)pyrrolidine-2-carboxamide, (S)-1-(4-fluorophenylsulfonyl)-N-((4-isobutoxypyridin-2-yl)methyl) pyrrolidine-2-carboxamide, (S)-1-(4-fluorophenylsulfonyl)-N-((4-(isobutylthio)pyridin-2-yl)methyl) pyrrolidine-2-carboxamide, (S)-1-(3,4-difluorophenylsulfonyl)-N-((4-(2,2,2-trifluoroethoxy) pyridin-2-yl)methyl)pyrrolidine-2-carboxamide, (S)-1-(3,5-difluorophenylsulfonyl)-N-((4-(2,2,2-trifluoroethoxy) pyridine-2-yl)methyl)pyrrolidine-2-carboxamide, (2S,4R)-N-(4-cyano-3-propylbenzyl)-4-fluoro-1-(phenylsulfonyl) pyrrolidine-2-carboxamide, (S)-N-(4-cyano-3-isopropylbenzyl)-1-(phenylsulfonyl)pyrrolidine-2-carboxamide, (2S,4R)-4-fluoro-1-(4-fluorophenylsulfonyl)-N-((2-(isobutylthio) pyridin-4-yl)methyl)pyrrolidine-2-carboxamide, (S)-N-(4-bromobenzyl)-1-(4-fluorophenylsulfonyl)pyrrolidine-2-carboxamide, (S)-N-(4-ethylbenzyl)-1-(4-fluorophenylsulfonyl)pyrrolidine-2-carboxamide or (S)-1-(4-fluorophenylsulfonyl)-N-(3-(propylamino)benzyl)pyrrolidine-2-carboxamide.

13. A compound according to any one of claims 1 to 12 for use as a medicament.

14. A compound according to any one of claims 1 to 12 for use in the treatment of a disorder, condition or disease mediated by TRPA1 receptor activity.

15. A compound according to claim 14, wherein the disorder, condition or disease is neuropathic pain, pain in diabetic polyneuropathy, postoperative pain, cancer pain, migraine, asthma, COPD, cough, pain in osteoarthritis, pain in rheumatoid arthritis or inflammatory bowel disease.
